# EUROPEAN PATENT APPLICATION

(11) **EP 4 378 538 A1**
(43) Date of publication of application: **05.06.2024**
(21) Application number: 21951345.4
(22) Date of filing: 30.07.2021
(51) Int. Cl.: A62B 23/06, A61M 15/00, A61M 15/08, A61M 16/00

(54) **NASAL FILTER**

(71) Applicant: Wang, Lei, Beijing 100085 (CN)
(72) Inventor: Wang, Lei, Beijing 100085 (CN)
(74) Representative: Bryn Aarflot AS
(86) International application number: PCT/CN2021/109604
(87) International publication number: WO 2023/004749

(57) **Abstract**

A nasal filter for filtering suspended particulate matters, suspended droplets, and harmful gases in air, and for waterproofing. The nasal filter (1) is arranged within nasal passages, and comprises a connecting beam (2) and two filtering mechanisms; two ends of the connecting beam (2) are respectively integrally or detachably connected to the two filtering mechanisms arranged symmetrically in the nasal passages (95) on two sides; the connecting beam (2) semi-surrounds a lower end of a nasal septum (91), and comprises an intermediate portion (21) positioned below the nasal septum (91), and two connecting arms (22) arranged symmetrically on two sides of the nasal septum (91); the intermediate portion (21) is positioned between the two connecting arms (22); the two connecting arms (22) are connected to the two filtering mechanisms arranged symmetrically in the two nasal passages (95); and each filtering mechanism comprises an outer frame (3), a filter plate (4), and a bracket (5).

## Description

### TECHNICAL FIELD

The present invention belongs to the technical field of medical devices, and specifically relates to a nasal filter, in particular to a nasal filter arranged within nasal passages.

### BACKGROUND

People often use masks to filter pollen, dust, dander, spores, respiratory droplets, tar and other suspended particulates and suspended droplets, as well as benzene, formaldehyde and other harmful gases in air to prevent and treat allergic rhinitis, allergic asthma, influenza and other diseases, and to avoid being harmed by atmospheric pollution. However, wearing a mask is not aesthetically pleasing, affects daily communication, and is liable to leakage at edges. It also affects a wearer's view from below. In cold weather, a mask also causes fog and frost to condense on glasses, thereby further obscuring the wearer's vision. Since inhalable particulate matters and droplets enter the human body mainly through nasal passages, arranging a nasal filter in the nasal passages can filter out most of the inhalable suspended particulate matters and droplets. Hidden in the nasal passages, the nasal filter does not affect the wearer's outside image and daily communication, does not block his view, and is not liable to leakage at edges, so it may be an alternative to a mask to some degree.

The prior art provides various nasal filters, which can mainly be grouped into the following types:
1. A membrane filter material covers nostrils and is fixed to the face by bonding or the like. Since this type of nasal filter is arranged outside the nasal passages of a wearer, it affects the outside image of the wearer and degrades user experience. In addition, due to a small nostril area, it is not possible to take into account both filterability and air permeability for the membrane filter material covering the nostrils. Furthermore, the fixation mode of this type of nasal filter is not reliable, is prone to escape and brings a risk of inhalation.
2. A filter material such as sponge is made into a cylindrical or hemispherical structure and arranged inside the nasal passages. This type of nasal filter is simple in structure and convenient to use. However, substances filtered out contact the skin or mucosa on inner walls of the nasal passages, thereby causing inflammation of the nasal passages. In addition, in order to hold the nasal filter inside the nasal passages of a wearer, a filter layer of this type of nasal filter is usually thick, thus reducing its air permeability.
3. A cylindrical or hemispherical filter material is wrapped inside a tubular outer frame, and an end of the tubular outer frame close an inner side of the nasal passage is provided with a blocking net to avoid the filter material being inhaled, and two tubular outer frames are connected by a connecting band, which is located under a nasal septum when worn. Such a nasal filter can avoid the contact of filtered substances with the nasal passages, but its air permeability is poor due to a thick filter material, a small air permeable area and blocking by the tubular outer frame and the consider blocking net.

In summary, as the space of nasal passages is narrow, the air permeable area of a nasal filter is much smaller than that of a mask. For the existing nasal filters, it is difficult to take into account both filtration and air permeability. In addition, filtering in an exhalation phase is not practically useful, and increases expiratory resistance and degrades user experience. However, unidirectional filtration has not yet been achieved in the existing nasal filters.

In addition, when a wearer performs water sports, unpurified water often splashes or gushes into the nasal passages, causing rhinitis and nasosinusitis, and the wearer usually uses a traditional swimming nasal clip to close his nasal passages. However, the use of a swimming nasal clip has the following limitations:
First, it affects the wearer's outside image.
Second, wearing for a long time can damage the skin of the nasal part of the wearer.
Third, different from underwater sports such as swimming and diving, water surface sports such as surfing and boating only need to shield splashed water droplets. Using a swimming nasal clip to close the nasal passages for a long time reduces sports fun for the wearer, and thereby degrades the wearer's user experience.

In summary, the prior art cannot provide a universal nasal filter that is aesthetically pleasing and capable of switching between water surface sports such as surfing and underwater sports such as swimming.

### SUMMARY OF THE INVENTION

To deal with the above-mentioned shortcomings of the prior art, the present invention proposes a nasal filter, specifically relating to a nasal filter arranged within nasal passages. The nasal filter is arranged within nasal passages, and includes: a connecting beam and two filtering mechanisms; two ends of the connecting beam are integrally or detachably connected to the two filtering mechanisms arranged symmetrically in the nasal passages on two sides;
the connecting beam semi-surrounds a lower end of a nasal septum, and includes an intermediate portion positioned below the nasal septum, and two connecting arms arranged symmetrically on two sides of the nasal septum; the intermediate portion is positioned between the two connecting arms; the two connecting arms are connected to the two filtering mechanisms arranged symmetrically in the two nasal passages;
each filtering mechanism includes an outer frame, a filter plate, and a bracket;
the outer frame includes an annular frame and at least one spoke, the annular frame being a hollow ring-like, elliptical columnar or tubular structure open at two ends and forming a side wall of the outer frame; when worn, an axis of the annular frame is parallel to a nasal passage axis, an outer circumferential wall of the annular frame is fit to an inner wall of the nasal passage, and an inner cavity of the annular frame is through in the direction of the nasal passage axis; the spoke is a strip-like structure, two ends of which are integrally or detachably connected to the top of the annular frame, and the spoke forms the top of the outer frame;
the sheet-like filter plate is provided below the top of the outer frame; specifically, the filter plate is positioned below the spoke, and completely covers an opening above the annular frame; the filter plate includes a fixed portion and at least one openable-closable portion, the fixed portion being located in the middle of the filter plate, the openable-closable portion being located at an edge part of the filter plate, and the openable-closable portion being not constrained by the bracket below; and in an exhalation state, an expiratory airflow pushes the openable-closable portion to switch from a closed state to an open state, so that the filtering mechanism has a unidirectional filtering function; and
the bracket is sleeved in an inner cavity of the outer frame, and the bracket is positioned below the filter plate, and the outer frame and the bracket constrain and fix the filter plate from above and below.

As one of improvements of the above technical solution, the outer frame further includes a sealing edge; the sealing edge is a straight-line, polyline or arc-line-shaped strip-like structure, which is provided along an edge of the top of the annular frame; the sealing edge is integrally connected to the top of the annular frame, and a main body thereof protrudes from an inner wall of the annular frame toward the direction of the axis of the annular frame; and two ends of the sealing edge are integrally connected to roots of the spoke.

As one of improvements of the above technical solution, at least one fixing rail is also longitudinally provided at an inner side of the outer frame; the fixing rail is a prism or a semi-circular cylinder whose axis is parallel to the axis of the annular frame; a side surface of the fixing rail is integrally connected to an inner wall of the annular frame, and a main body thereof protrudes from the inner wall of the annular frame toward the axis of the annular frame; and correspondingly, the filter plate is longitudinally provided with groove-like clamping slots on two sides to match therewith, and the bracket is longitudinally provided with groove-like fixing slots on two sides to match therewith.

As one of improvements of the above technical solution, the outer frame is also provided with at least one strip-like or block-like clamping tooth; the clamping tooth is provided on an inner wall close a lower end of the annular frame, with a side surface thereof being integrally connected to an inner wall of the annular frame, and a main body thereof protruding from the inner wall of the annular frame toward the direction of the axis of the annular frame.

As one of improvements of the above technical solution, the filter plate is an elastomer.

As one of improvements of the above technical solution, the filter plate 4 includes a filter layer body and an elastic layer stacked with the filter layer body.

As one of improvements of the above technical solution, the filter layer body is a stacked multi-layer structure, which includes a plurality of layer structures made of filter materials with different filtering functions or filtering efficiencies.

As one of improvements of the above technical solution, the filter plate is a convex-face or serrated-face sheet.

As one of improvements of the above technical solution, the openable-closable portion is inclined downwards in a direction away from the nasal passage axis.

As one of improvements of the above technical solution, an angle of inclination of the openable-closable portion inclined downwards in the direction away from the nasal passage axis increases progressively.

As one of improvements of the above technical solution, the bracket includes a base and at least one support beam;
the base is a hollow ring-like, elliptical columnar or tubular structure open at two ends, and is located in a lower middle part of the bracket, and after assembly, an axis of the base is parallel to the axis of the annular frame, and an outer circumferential wall thereof is tightly engaged with an inner wall of the annular frame; an inner cavity of the base is through in the direction of the nasal passage axis;
the support beam is a strip-like structure, the appearance of which is a straight-line, polyline, arc-line, arch, downward-open rectangle or downward-open trapezoid shape; the support beam is located in an upper middle part of the bracket; the support beam has two ends connected to the top of the base or other remaining support beams, and together with the spoke, clips and fixes the fixed portion of the filter plate in both directions from below and above; and no support beam is provided below the openable-closable portion.

As one of improvements of the above technical solution, the bracket further includes at least one side wall; the side wall is a vertical flat plate wall or arc-shaped plate wall-like structure, which is located in the upper middle part of the bracket; the side wall has a bottom end fixed to the base, a top end located directly below the corresponding sealing edge, and two sides connected to the support beam; and the side wall and the sealing edge clip and fix an edge part of the fixed portion of the filter plate in both directions from below and above.

As one of improvements of the above technical solution, the bracket is also provided with at least one snap; the snap is a vertical strip-like or sheet-like structure; and the snap has an upper end integrally connected to an edge part of a lower surface of the base, and a main body protruding downward from the lower surface of the base.

As one of improvements of the above technical solution, the bracket is also provided with at least one elastic carrier; the elastic carrier includes at least one elastic strip and at least one optional carrier strip; the elastic strip has one end integrally or detachably fixed to the support beam, a main body extending from the fixed part toward a direction away from the nasal passage axis, and the other end not fixed; the carrier strip is a transversely arranged strip, which is fixed to the elastic strip; and after assembly, the elastic carrier is positioned below the openable-closable portion, and an upper surface thereof is tightly fit to or close to a lower surface of the openable-closable portion.

As one of improvements of the above technical solution, the filtering mechanism further includes a filter body; and after assembly, the filter body is arranged in an inner cavity of the bracket.

As one of improvements of the above technical solution, the filter body is an elastomer.

As one of improvements of the above technical solution, the filter body is a stacked multi-layer structure, which includes a plurality of layer structures made of filter materials with different filtering functions or filtering efficiencies.

As one of improvements of the above technical solution, the filtering mechanism further includes a waterproof stopper; the waterproof stopper includes a stopper body, at least one skirt and at least one connecting band;
the stopper body is a column structure, the shape and size of a transverse cross-section of the top of which are same as the shape and size of a transverse cross-section of an inner cavity at a lower end of the bracket or the annular frame, which enables the stopper body to be plugged into and close the lower end of the bracket or the annular frame; the skirt is a ring-like structure or discontinuous ring-like structure, which surrounds a lower part of the stopper body and is integrally connected to the stopper body; and the connecting band is a band-like structure with one end integrally or detachably connected to the waterproof stopper and the other end integrally or detachably connected to the connecting beam, the outer frame or the bracket.

As one of improvements of the above technical solution, the two filtering mechanisms each include a waterproof stopper, respectively, wherein one waterproof stopper is provided with at least one columnar storage plug integrally connected therewith and protruding from a lower surface thereof, and the other waterproof stopper is provided with at least one tubular storage slot integrally connected therewith and protruding from a lower surface thereof, the storage plug and the storage slot being provided in pair and being matched with each other; and
when the two waterproof stoppers are both in an open state, the two waterproof stoppers are placed opposite each other, the storage plug provided on the one waterproof stopper is inserted into the storage slot provided on the other waterproof stopper, and the former is tightly engaged with the latter, and by using friction therebetween, the two opposite waterproof stoppers are connected under the nasal septum.

As one of improvements of the above technical solution, the connecting beam applies a clipping force to the nasal septum; and the clipping force is applied directly to two sides of the nasal septum by the two connecting arms of the connecting beam, or after being conducted by the two connecting arms of the connecting beam to the corresponding filtering mechanisms, the clipping force is applied to the two sides of the nasal septum by the corresponding filtering mechanisms.

Compared with the prior art, the two present invention has the following beneficial effects:
1. The nasal filter of the present invention can achieve a variety of uses by providing different filter plates, not only can filter out suspended particulate matters, suspended droplets and harmful gases in air, but also can filter out water droplets splashed during surfing or boating. In addition, the nasal filter of the present invention is also provided with an openable-closable waterproof stopper to close the nasal passage. Compared with a traditional swimming nasal clip, the nasal filter of the present invention can be adapted to different water sports. When performing water surface sports such as surfing and boating, a wearer can open the waterproof stopper, such that the wearer can breathe normally most of the time, and the filter plate can shield occasionally splashed water droplets in time. When performing underwater sports such as swimming and diving, the wearer can close the waterproof stopper to avoid water entering the nasal passage. Compared with a swimming nasal clip, the nasal filter of the present invention also has the advantages of being aesthetically pleasing, not harming the skin of the outer side of the nasal part, having low swimming resistance, etc. and brings better user experience.
2. The nasal filter of the present invention has more excellent air permeability. On the one hand, the unidirectional filtering function of the filtering mechanism significantly reduces expiratory resistance. On the other hand, the nasal filter of the present invention provides a specific implementation of increasing the air permeable area and air permeability by means of the convex-face and serrated-face filter plates.
3. The nasal filter of the present invention allows convenient replacement of the filter plate. On the one hand, the wearer can switch between multiple uses by using different types of filter plates. On the other hand, by replacing an old filter plate with a new identical one, a main body of the nasal filter can be used for a long time, which improves the economy of the nasal filter.
4. The nasal filter of the present invention has a compact and solid structure.
   First, by means of the clamping tooth and the snap, the wearer can conveniently install and remove the bracket and the filter plate.
   Second, by providing the fixing rail, deviation of the bracket and the filter plate inside the outer frame is effectively avoided.
   Third, by providing the storage devices of the waterproof stoppers, shaking of the waterproof stoppers in the open state can be effectively avoided, and the comfort of use is improved.
5. The nasal filter of the present invention may also be provided with a filter body therein. The filter body can be a porous material such as activated carbon sponge, for filtering suspended particulate matters, suspended droplets or harmful gases in air, thereby further increasing the filtering effectiveness and application range of the nasal filter.

### BRIEF DESCRIPTION OF THE DRAWINGS

Fig. 1A is a side view of a human nasal part;
Fig. 1B is a side sectional view of a human nasal part;
Fig. 1C is a bottom view of a human nasal part;
Fig. 2 is a three-dimensional structural diagram of a first preferred embodiment of a nasal filter of the present invention;
Fig. 3 is an assembly diagram of a first preferred embodiment of a nasal filter of the present invention;
Fig. 4 is a top view of a first preferred embodiment of a nasal filter of the present invention;
Fig. 5 is a rear view of a first preferred embodiment of a nasal filter of the present invention;
Fig. 6 is a side view of a first preferred embodiment of a nasal filter of the present invention;
Fig. 7 is a bottom view of a first preferred embodiment of a nasal filter of the present invention;
Fig. 8A is a side view of a first preferred embodiment of a nasal filter of the present invention mounted to a nasal part of a wearer;
Fig. 8B is a bottom view of a first preferred embodiment of a nasal filter of the present invention mounted to a nasal part of a wearer;
Fig. 9 is a three-dimensional structural diagram of an outer frame in a first preferred embodiment of a nasal filter of the present invention;
Fig. 10 is a bottom view of an outer frame in a first preferred embodiment of a nasal filter of the present invention;
Fig. 11 is a top view of an outer frame provided with a fourth spoke in a first preferred embodiment of a nasal filter of the present invention;
Fig. 12 is a top view of an outer frame provided with a small spoke in a first preferred embodiment of a nasal filter of the present invention;
Fig. 13 is a side view of a filter plate of a first preferred embodiment of a nasal filter of the present invention, a filter layer body of the filter plate being a single-layer structure;
Fig. 14 is a side view of a filter plate of a first preferred embodiment of a nasal filter of the present invention, a filter layer body of the filter plate being a double-layer structure;
Fig. 15 is a side view of a filter plate of a first preferred embodiment of a nasal filter of the present invention, the filter plate including a filter layer body and an elastic layer;
Fig. 16 is a side view of a filter plate and a bracket in a first preferred embodiment of a nasal filter of the present invention;
Fig. 17 is a top view of a filter plate and a bracket in a first preferred embodiment of a nasal filter of the present invention;
Fig. 18 is a schematic diagram of a first preferred embodiment of a nasal filter of the present invention having a unidirectional filtering function;
Fig. 19A is a top-view diagram of a first distribution mode of an openable-closable portion of a filter plate of a nasal filter of the present invention;
Fig. 19B is a top-view diagram of a second distribution mode of an openable-closable portion of a filter plate of a nasal filter of the present invention;
Fig. 19C is a top-view diagram of a third distribution mode of an openable-closable portion of a filter plate of a nasal filter of the present invention;
Fig. 20A is a lateral diagram of a filter plate of a nasal filter of the present invention with an openable-closable portion in a horizontal form;
Fig. 20B is a lateral diagram of a filter plate of a nasal filter of the present invention with an openable-closable portion in an inclined form;
Fig. 21A is a three-dimensional structural diagram of a bracket in a first preferred embodiment of a nasal filter of the present invention;
Fig. 21B is a three-dimensional structural diagram of a bracket, side walls of which are not shown, in a first preferred embodiment of a nasal filter of the present invention;
Fig. 22 is a top view of a bracket in a first preferred embodiment of a nasal filter of the present invention;
Fig. 23 is a bottom view of a bracket in a first preferred embodiment of a nasal filter of the present invention;
Fig. 24 is a three-dimensional structural diagram of a bracket provided with a fourth support beam in a first preferred embodiment of a nasal filter of the present invention;
Fig. 25 is a three-dimensional structural diagram of a bracket provided with an elastic carrier in a first preferred embodiment of a nasal filter of the present invention;
Fig. 26 is a top view of a bracket provided with an elastic carrier in a first preferred embodiment of a nasal filter of the present invention;
Fig. 27 is a side view of a filter plate and a bracket provided with an elastic carrier in a first preferred embodiment of a nasal filter of the present invention;
Fig. 28 is a three-dimensional structural diagram of a bracket and a filter body in first preferred embodiment of a nasal filter of the present invention provided with the filter body;
Fig. 29 is an assembly diagram of a bracket and a filter body in first preferred embodiment of a nasal filter of the present invention provided with the filter body;
Fig. 30 is a bottom view of a bracket and a filter body in first preferred embodiment of a nasal filter of the present invention provided with the filter body;
Fig. 31 is a front view of a first preferred embodiment of a nasal filter of the present invention provided with a waterproof stopper;
Fig. 32 is a bottom view of a first preferred embodiment of a nasal filter of the present invention provided with a waterproof stopper;
Fig. 33 shows a rear view of a filter plate, a bracket and a waterproof stopper in a first preferred embodiment of a nasal filter of the present invention provided with the waterproof stopper, the waterproof stopper being in a closed state;
Fig. 34 is a three-dimensional structural diagram of a bracket and a waterproof stopper in a first preferred embodiment of a nasal filter of the present invention provided with the waterproof stopper, the waterproof stopper being in an open state and stored;
Fig. 35 is a front view of other specific embodiments of a nasal filter of the present invention provided with a waterproof stopper, wherein an outer frame and a connecting beam on one side are removed;
Fig. 36A is a front view of a bracket and a waterproof stopper in a first preferred embodiment of a nasal filter of the present invention provided with the waterproof stopper, the waterproof stopper being in an open state and not stored;
Fig. 36B is a front view of a bracket and a waterproof stopper in a first preferred embodiment of a nasal filter of the present invention provided with the waterproof stopper, the waterproof stopper being in an open state and stored;
Fig. 37 is a three-dimensional structural diagram of a second preferred embodiment of a nasal filter of the present invention;
Fig. 38 is a three-dimensional structural diagram of an outer frame in a second preferred embodiment of a nasal filter of the present invention;
Fig. 39 is a bottom view of an outer frame in a second preferred embodiment of a nasal filter of the present invention;
Fig. 40 is a side view of a filter plate in a second preferred embodiment of a nasal filter of the present invention;
Fig. 41 is a three-dimensional structural diagram of a bracket in a second preferred embodiment of a nasal filter of the present invention; and
Fig. 42 is a three-dimensional structural diagram of a second preferred embodiment of a nasal filter of the present invention provided with a protective cover.

**Reference numerals:**

| | |
|---|---|
| 1, Nasal filter | 2, Connecting beam |
| 3, Outer frame | 4, Filter plate |
| 5, Bracket | 6, waterproof stopper |
| 7, Filter body | 9, Nasal part |
| 11, Forward direction of nasal part | 12, Rearward direction of nasal part |
| 13, Upward direction of nasal part | 14, Downward direction of nasal part |
| 21, Intermediate portion | 22, Connecting arm |
| 31, Annular frame | 32, Spoke |
| 33, Sealing edge | 34, fixing rail |
| 35, Clamping tooth | 36, Protective cover |
| 37, Small spoke | |
| 41, Filter layer body | 42, Elastic layer |
| 43, Clamping slot | 44, Fixed portion |
| 45, openable-closable portion | 45', Openable-closable portion in open state |
| 51, Base | 52, Side wall |
| 53, Support beam | 54, Snap |
| 55, Fixing slot | 56, Elastic carrier |
| 57, Fixing tooth | |
| 61, Stopper body | 62, Skirt |
| 63, Storage plug | 64, Storage slot |
| 65, Connecting band | |
| 91, Nasal septum | 92, Nasal wing |
| 93, Nasal bridge | 94, Nostril |
| 95, Nasal passage | 96, Inner wall of nasal passage |
| 97, Nasal passage axis | 98, Nasal cavity |
| 99, Nasal base | |
| 321, First spoke | 322, Second spoke |
| 323, Third spoke | 324, Fourth spoke |
| 325, Fifth spoke | 326, Sixth spoke |
| 331, First sealing edge | 332, Second sealing edge |
| 333, Third sealing edge | 334, Fourth sealing edge |
| 335, Fifth sealing edge | 336, Sixth sealing edge |
| 411, First filter layer body | 412, Second filter layer body |
| 521, First side wall | 522, Second side wall |
| 523, Third side wall | 524, Fourth side wall |
| 531, First support beam | 532, Second support beam |
| 533, Third support beam | 534, Fourth support beam |
| 535, Fifth support beam | 536, Sixth support beam |
| 561, Elastic strip | 562, Carrier strip |

### DETAILED DESCRIPTION

Now, the present invention is further described in conjunction with the accompanying drawings.

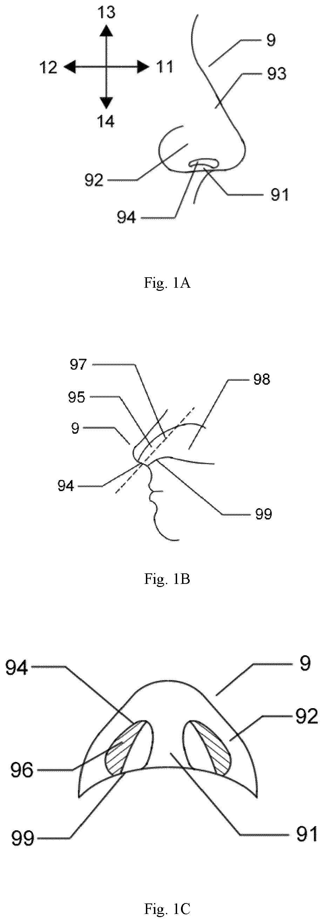

As shown in Fig. 1A, directions for a nasal part described in the present invention are: a forward direction 11 of the nasal part, a rearward direction 12 of the nasal part, an upward direction 13 of the nasal part, and a downward direction 14 of the nasal part.

As shown in Fig. 1B, in a nasal passage 95, a side of an object close to a nasal passage axis 97 is an "inner side", and a side away from the nasal passage axis 97 is an "outer side".

The term "connection" as used in the present invention means that two tangible objects are integrally or detachably connected together, and connection modes include but are not limited to: integrally forming, adhesive bonding, fusion splicing, plug-in connection, screw connection, and snap connection.

The term "axis" as used in the present invention refers to an axis of centrosymmetry of a tube ring or column. For a non-standard tube, ring or column, an axis of a standard tube, ring or column similar to it can be used as its approximate axis. For example, Fig. 1B depicts the nasal passage axis 97.

The term "transverse cross-section" as used in the present invention refers to a cross-section of the tube, ring or column perpendicular to its axis.

As shown in Figs. 1A, 1B and 1C, the nasal filter 1 of the present invention is applied to a human nasal part 9. The nasal part 9 is a visually visible facial protruding part, which includes: a nasal septum 91, nasal wings 92, a nasal bridge 93 and a nasal base 99, and two nasal passages 95 enclosed thereby. Outlets of the two nasal passages 95 are two nostrils 94. An inner wall 96 of a nasal passage is composed of inner side surfaces of the parts enclosing the nasal passage 95 as described above. In the present invention, a nasal passage 95 is limited to a nasal vestibule contained by the nasal part 9 and does not include a nasal cavity 98.

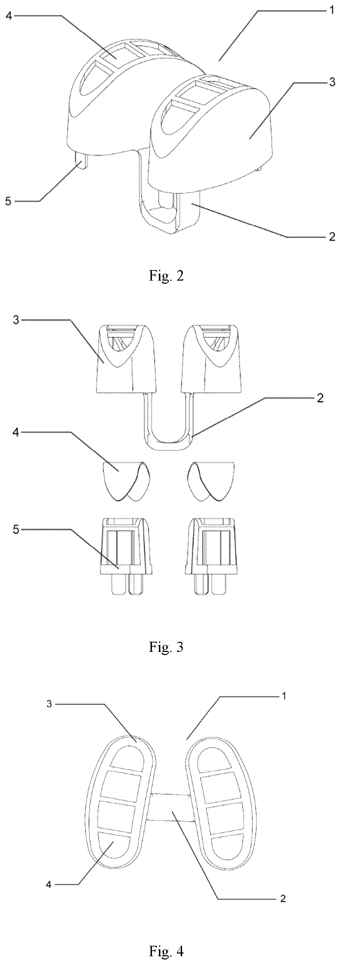

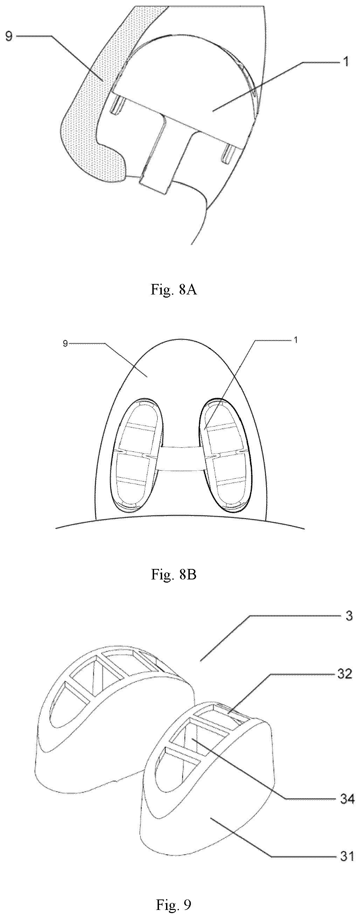

As shown in Fig. 2, the present invention provides a nasal filter 1 arranged within nasal passages 95. It includes a connecting beam 2 and two filtering mechanisms. Two ends of the connecting beam 2 are respectively integrally or detachably connected to the two filtering mechanisms that are arranged symmetrically. As shown in Figs. 8A and 8B, when worn, the two filtering mechanisms are respectively inserted into the two nasal passages 95 from the bottom up, and the connecting beam 2 semi-surrounds a lower end of a nasal septum 91.

As shown in Figs. 2, 5, 8A and 8B, the connecting beam 2 includes an intermediate portion 21 positioned below the nasal septum 91 and two connecting arms 22 positioned on two sides of the nasal septum 91. The intermediate portion 21 is positioned between the two connecting arms 22. The two connecting arms 22 are respectively connected to the two filtering mechanisms arranged symmetrically in the two nasal passages 95. When worn, the connecting beam 2 can avoid the filtering mechanisms being sucked in, and a wearer can also pull the connecting beam 2 to remove the nasal filter 1. Preferably, the connecting beam 2 is a U-shaped or C-shaped structure.

Preferably, the connecting beam 2 also provides a fixing function for the nasal filter 1. A minimum distance between its two connecting arms 22 is less than a width of the nasal septum 91 there, so that they apply a clipping force to the nasal septum 91. The clipping force is applied to the two sides of the nasal septum 91 directly by the two connecting arms 22 of the connecting beam 2, or is conducted by the two connecting arms 22 of the connecting beam 2 to the corresponding filtering mechanisms and then applied to the two sides of the nasal septum 91 by the outer frame 3. The above-mentioned clipping force enables the nasal filter 1 to clip and be fixed to the nasal septum 91.

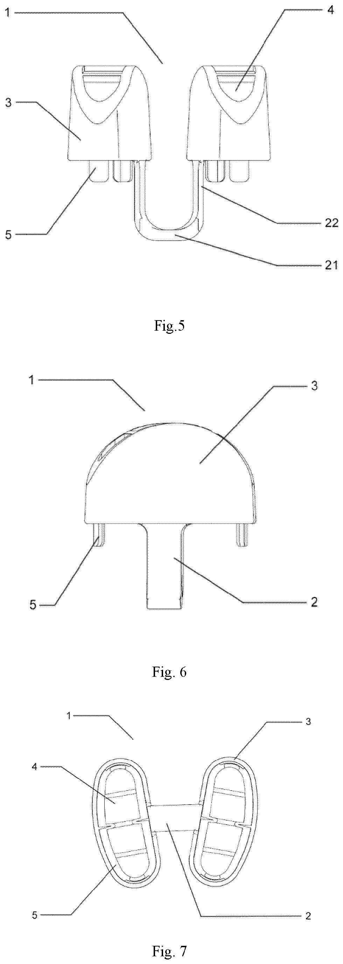

As shown in Figs. 2 and 5, the two filtering mechanisms are symmetrically connected to the connecting beam 2, and each filtering mechanism includes an outer frame 3, a filter plate 4 and a bracket 5. As shown in Fig. 3, in assembly, first the filter plate 4 is mounted into the outer frame 3 from the bottom up, and then the bracket 5 is mounted and fixed to an inner side of the outer frame 3 from the bottom up. After assembly, the outer frame 3 and the bracket 5 sleeved in its inner cavity fix the filter plate 4 in both directions from above and below respectively, i.e. the filter plate 4 is positioned between the outer frame 3 and the bracket 5.

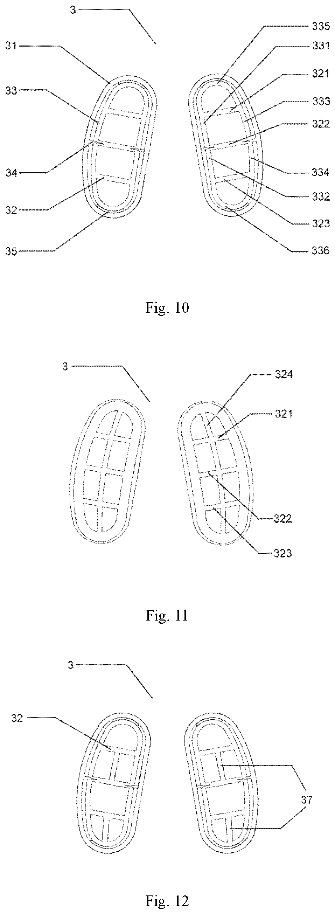

As shown in Figs. 9 and 10, the outer frame 3 includes an annular frame 31 and at least one spoke 32. The annular frame 31 forms a side wall of the outer frame 3, and the spoke 32 forms the top of the outer frame 3.

As shown in Figs. 9 and 10, the annular frame 31 is a hollow ring-like, elliptical columnar or tubular structure open at two ends. When worn, an axis of the annular frame 31 is parallel to a nasal passage axis 97, and an outer circumferential wall of the annular frame 31 is fit to or tightly engaged with an inner wall 96 of the nasal passage, and their interaction forces fix the nasal filter 1 in the nasal passage 95. An inner cavity of the annular frame 31 is through in the direction of the nasal passage axis 97, such that inspiratory and expiratory airflows can pass smoothly through the annular frame 31. Walls of the annular frame 31 are thin to reduce their resistance to breathing. As shown in Figs. 5 and 9, preferably, the annular frame 31 is an irregular truncated cone with a small top and a large bottom, so as to better match the shape of the nasal passage 95.

As shown in Fig. 9, the spoke 32 is a strip-like structure, the appearance of which is a straight-line, polyline, arc-line, arch, or other common shape or irregular shape. The spoke 32 is provided on the top of the outer frame 3, and two ends thereof are integrally or detachably connected to different positions on an inner wall or upper surface of the top of the annular frame 31. The spoke 32 and a support beam 53 of the bracket 5 clip and are fixed to the filter plate 4 in both directions from above and below respectively. The spoke 32 also constrain the filter plate 4 and the bracket 5 below the outer frame 3 to avoid a risk of being sucked in. As shown in Fig. 10, a first spoke 321, a second spoke 322 and a third spoke 323 are all straight-line strip-like structures, and the first spoke 321, the second spoke 322 and the third spoke 323 are arranged parallel to each other at the top of the outer frame 3. As shown in Fig. 11, preferably, a first spoke 321, a second spoke 322 and a third spoke 323 are arranged parallel to each other, and a fourth spoke 324 vertically passes through the first spoke 321, the second spoke 322 and the third spoke 323 to form a grid-like structure, i.e., a plurality of spokes 32 can cross each other and their intersecting parts can be fused integrally to form a grid-like structure. As shown in Fig. 12, preferably, the top of the outer frame 3 is also provided with a small spoke 37. Both ends of the small spoke 37 are connected between a plurality of spokes 32 arranged in parallel, or one end of the small spoke 37 is connected to a spoke 32 and the other end thereof is connected to the inner wall of the top of the annular frame 31. The small spoke 37 is used to assist the spokes 32 to achieve a function of constraining and fixing the filter plate 4. In other specific embodiments, the spokes 32 may also be arranged non-parallel to each other, e.g., arranged in a crossed manner to form a grid-like structure. One end of the small spoke 37 can be connected to a spoke 32, and the other end thereof may also be connected to the upper surface of the top of the annular frame 31.

As shown in Fig. 10, preferably, the outer frame 3 further includes at least one sealing edge 33. The sealing edge 33 is a strip-like structure, the appearance of which is a straight-line, polyline, arc-line, arch, or other common shape or irregular shape. The sealing edge 33 is provided along an edge of the top of the annular frame 31, for avoiding leakage at an edge part where the filter plate 4 contacts the annular frame 31. An outer side surface or a lower surface of the sealing edge 33 is integrally connected to the top of the annular frame 31, and a main body thereof protrudes from an inner wall of the annular frame 31 toward the direction of the axis of the annular frame 31. Two ends of the sealing edge 33 are integrally connected to roots of the spokes 32. As shown in Fig. 10, a total of 6 sealing edges, namely a first sealing edge 331, a second sealing edge 332, a third sealing edge 333, a fourth sealing edge 334, a fifth sealing edge 335 and a sixth sealing edge 336, have both ends integrally connected to roots of the spokes 32 and completely cover contact parts of the filter plate 4 and the annular frame 31.

As shown in Figs. 9 and 10, preferably, the outer frame 3 is also longitudinally provided with at least one fixing rail 34 extending from the top to the lower part thereof, to fix the filter plate 4 and the bracket 5. The fixing rail 34 is a prism or a semi-circular cylinder whose axis is parallel to the axis of the annular frame 31. A side surface of the fixing rail 34 is integrally connected to the inner wall of the annular frame 31, and a main body thereof protrudes from the inner wall of the annular frame 31 toward the axis of the annular frame 31. As shown in Figs. 3, 13, 21A and 21B, a side face of the filter plate 4 is longitudinally provided with a groove-shaped clamping slot 43, and a side face of the bracket 5 is longitudinally provided with a groove-shaped fixing slot 55. In assembly, the clamping slot 43 and the fixing slot 55 are aligned with the fixing rail 34, and then the filter plate 4 and the bracket 5 are successively put into the outer frame 3 along the fixing rail 34. After assembly, the clamping slot 43 and the fixing slot 55 are tightly engaged with the fixing rail 34, thereby avoiding lateral or rotational displacement of the filter plate 4 and the bracket 5 inside the outer frame 3.

As shown in Fig. 10, preferably, the inner wall of the annular frame 31 is also provided with at least one strip-like or block-like clamping tooth 35, for fixing the bracket 5 to avoid it escaping from the outer frame 3. The clamping tooth 35 is provided on the inner wall close a lower end of the annular frame 31, with a side surface thereof being integrally connected to the inner wall of the annular frame 31, and a main body thereof protruding from the inner wall of the annular frame 31 toward the direction of the axis of the annular frame 31. In assembly, after the bracket 5 is inserted into the cavity of the annular frame 31 from its bottom end, an outer circumferential wall of a base 51 is tightly fit to the inner wall of the annular frame 31, and an upper surface of the clamping tooth 35 clamps an edge of a lower surface of the base 51 so that the bracket 5 cannot escape toward the underside of the outer frame 3.

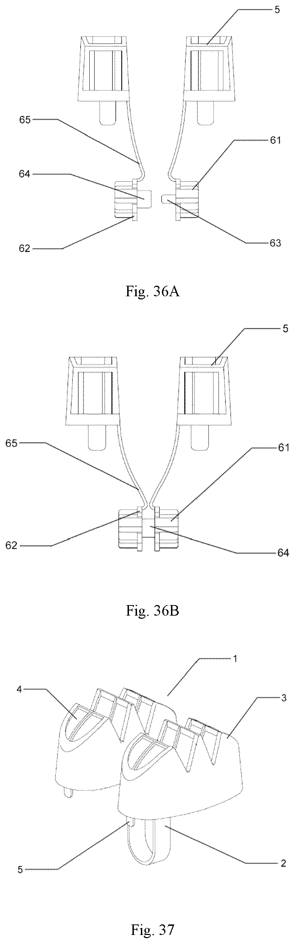

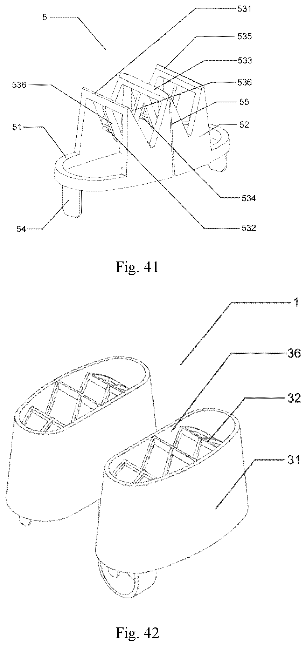

As shown in Figs. 2 and 37, preferably, the top of the outer frame 3 is configured as a convex-face or serrated-face structure to match the filter plate 4 of the same shape to increase a filtering area and improve air permeability. As shown in Fig. 42, as an optimization, in order to avoid that a sharp part of the top of the annular frame 31 injures the inner wall 96 of the nasal passage in some specific embodiments, a ring-like protective cover 36 is also sleeved at an outer periphery of the outer frame 3. The protective cover 36 surrounds an upper part of the outer frame 3 from an outer side, with its lower end being connected to an upper end of the annular frame 31, and an upper end of the protective cover 36 is not lower than the highest point of the annular frame 31.

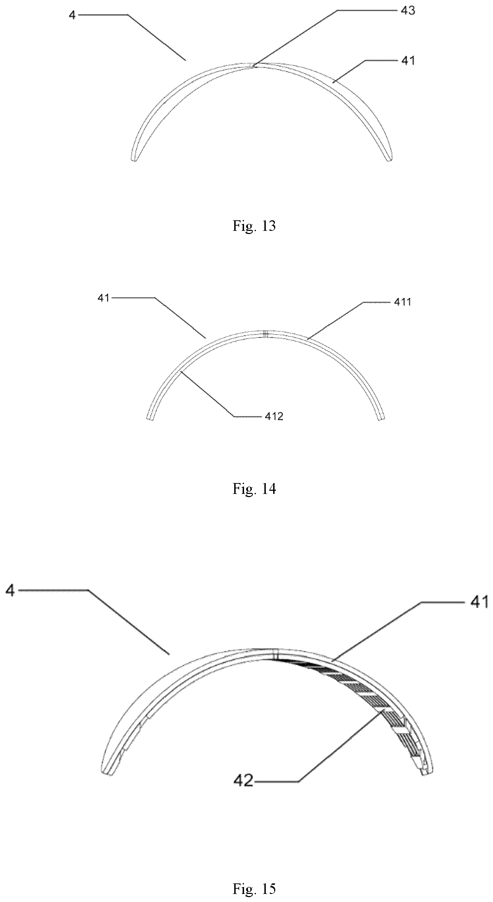

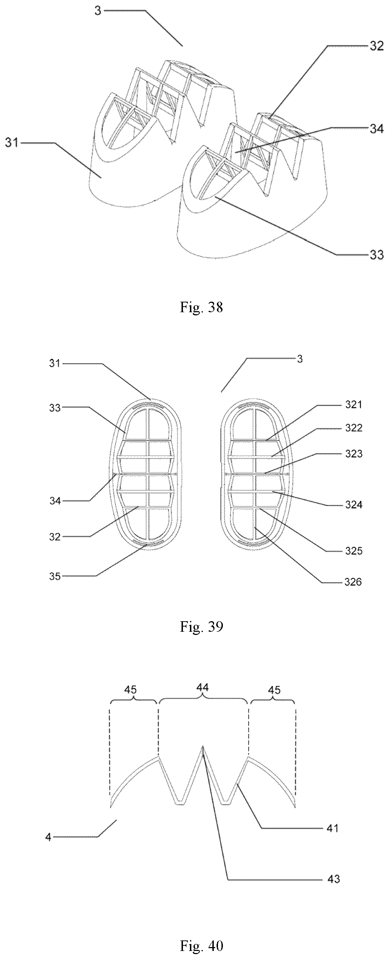

As shown in Figs. 2, 3 and 13, the filter plate 4 is a sheet, which is positioned below the top of the outer frame 3. Specifically, the filter plate 4 is positioned below the spoke 32 and is fixed between the outer frame 3 and the bracket 5. In a natural state, the filter plate 4 completely covers an opening above the annular frame 31, and an upper surface and a side face thereof are tightly fit to the inner wall of the outer frame 3, so that an airtight state is maintained between the filter plate 4 and the outer frame 3. As shown in Figs. 13 and 40, preferably, the filter plate 4 is a convex-face or serrated-face sheet, which can significantly increase an air permeable area and improve the air permeability and comfort of the nasal filter 1 compared with a planar sheet. As shown in Fig. 13, preferably, the side face of the filter plate 4 is also longitudinally provided with at least one groove-like clamping slot 43, and the clamping slot 43 cooperates with the fixing rail 34 on the outer frame 3 to fix the filter plate 4. Preferably, the filter plate 4 is an elastomer to assist the filtering mechanism with a unidirectional filtering function.

As shown in Fig. 13, the filter plate 4 includes a filter layer body 41 for filtering particulate matters, droplets, splashing water droplets or harmful gases in air. In the case the filter layer body 41 is made of a filter material such as non-woven fabric, filter cotton, melt-blown cloth, artificial sponge, a fine metal net or a polyester fiber net, it can filter out suspended particulate matters or suspended droplets in air. In the case the filter layer body 41 is made of a filter material such as activated carbon sponge or activated carbon filter cotton, it can filter out harmful gases in air. In the case the filter layer body 41 is made of a filter material containing a disinfecting or sterilizing substance such as nano-silver, it can kill bacteria or viruses attached to suspended particulate matters or suspended droplets in air. In the case the filter layer body 41 is made of a metal net or a polyester fiber net with an appropriate pore size, it can block water droplets splashed in surfing, boating and other surface water sports, by using surface tension of water, to avoid the water droplets being splashed into the nasal passages to cause rhinitis or nasosinusitis.

As shown in Fig. 14, preferably, the filter layer body 41 can be a stacked double-layer structure, containing a first filter layer body 411 and a second filter layer body 412 with different functions, which may or may not be bonded to each other. The first filter layer body 411 may be made of a strong or medium-effect filter material, and the second filter layer body 412 may be made of a primary-effect filter material, an activated carbon filter material, a nano-silver filter material, or the like. New filtration requirements can be met by using the two filter layers in combination. As an optimization, the filter layer body 41 can be a stacked multi-layer structure, with each layer being made of one of the above-mentioned filter materials, and a plurality of filter layers are used in combination to meet higher filtration requirements.

As shown in Fig. 15, preferably, in the case the elasticity of the filter layer body 41 is not enough, the filter plate 4 is also provided with an elastic layer 42 to improve the elasticity of the filter plate 4, thereby assisting the filtering mechanism to achieve a unidirectional filtering function described below. The layer body 41 and the elastic layer 42 are stacked. In the case the elastic layer 42 is positioned below the filter layer body 41, they may be or may be not bonded to each other. In the case the elastic layer 42 is positioned above the filter layer body 41, they are bonded to each other, so that a resilience force of the elastic layer 42 can be applied to the filter layer body 41. The size of the elastic layer 42 can be equal to or less than that of the filter layer body 41, and the material of the elastic layer 42 can be a net, strip or sheet with elasticity.

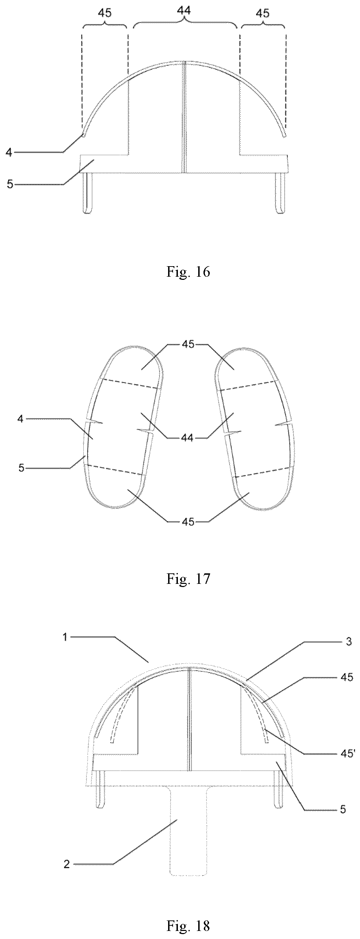

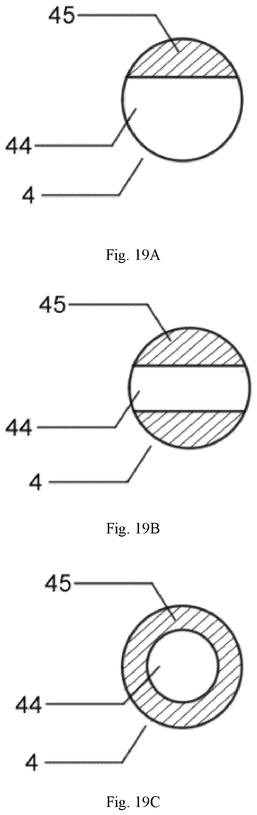

The filtering mechanism of the nasal filter 1 of the present invention has a unidirectional filtering function, specifically implemented as follows. As shown in Figs. 16, 17 and 18, the filter plate 4 includes a fixed portion 44 and at least one openable-closable portion 45. The fixed portion 44 is located in the middle of the filter plate 4. After assembly, the fixed portion 44 is tightly clipped by the outer frame 3 positioned above and the bracket 5 positioned below, such that the fixed portion 44 does not displace inside the outer frame 3. The openable-closable portion 45 is located at edge parts of two sides of the filter plate 4 and is constrained above by the outer frame 3 but is not constrained below by the bracket 5, so when the openable-closable portion 45 is in a closed state, once the openable-closable portion 45 is subjected to a top-down acting force, it can move downward and open from a closed position of being closely fit to a lower surface of the top of the outer frame 3. Preferably, the openable-closable portion 45 can be distributed on the filter plate 4 in various ways. For example, as shown in Fig. 19A, the openable-closable portion 45 is provided at an edge part of a side of the filter plate 4; as shown in Fig. 19B, the openable-closable portion 45 is provided at edge parts of two sides of the filter plate 4; as shown in Fig. 19C, the openable-closable portion 45 is provided at an annular edge part of the filter plate 4.

In summary, as shown in Fig. 18, the openable-closable portion 45 has two states as follows:
1. Closed state: In a natural state and an inhalation state, an upper surface and a side face of the openable-closable portion 45 are closely fit to the inner wall of the outer frame 3, and is in airtight state is maintained between the filter plate 4 and the outer frame 3, such that an inspiratory airflow can only pass through the filter plate 4, and the filtering mechanism performs the unidirectional filtering function.
2. Open state: In an exhalation state, under the action of a larger air pressure above, the openable-closable portion 45 moves downward to a position of an openable-closable portion 45' in the open state; at that point, a gap is generated between the filter plate 4 and the outer frame 3, and most of an expiratory airflow passes through the gap, and the filtering mechanism does not perform the filtering function; and after the end of the exhalation state, under the action of the resilience of the filter plate 4, the openable-closable portion 45 recovers to the closed state in the natural state.

The unidirectional filtering function has the beneficial effect that the expiratory airflow does not pass through the filter plate 4, but passes directly through the gap between the filter plate 4 and the outer frame 3, which greatly reduces exhalation resistance, thereby significantly improving the air permeability and comfort of the nasal filter 1 in the exhalation state.

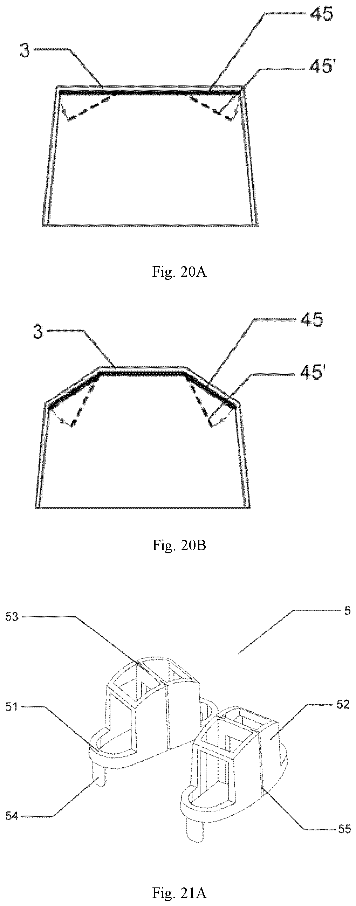

To better achieve the unidirectional filtering function, as shown in Figs. 16 and 40, preferably, the openable-closable portion 45 is inclined downwards in a direction away from the nasal passage axis 97 to increase the air permeation efficiency of the openable-closable portion 45 in the open state. As shown in Figs. 20A and 20B, when the openable-closable portion 45 is transformed from the closed state to the openable-closable part 45' in the open state, for the same angular displacement, a gap generated between the openable-closable part 45 in an inclined form and the outer frame 3 in Fig. 20B is significantly larger than a gap generated between the openable-closable part 45 in a horizontal form and the outer frame 3 in Fig. 20A. As a further optimization, an angle of downward inclination of the openable-closable portion 45 in the direction away from the nasal passage axis 97 increases progressively to further increase the air permeation efficiency in an exhalation phase.

As shown in Figs. 2, 3, 21A and 21B, the bracket 5 is sleeved in the inner cavity of the outer frame 3, and the bracket 5 is positioned below the filter plate 4, and includes a base 51 and at least one support beam 53.

As shown in Figs. 21A, 21B, 22 and 23, the base 51 is a hollow ring-like, elliptical columnar or tubular structure open at two ends, and is located in a lower middle part of the bracket 5. After assembly, an axis of the base 51 is parallel to the axis of the annular frame 31, and its outer circumferential wall is tightly engaged with the inner wall of the annular frame 31, and their interaction forces fix the bracket 5 in the inner cavity of the outer frame 3. An inner cavity of the base 51 is through in the direction of the nasal passage axis 97, and inspiratory and expiratory airflows can pass smoothly through its inner cavity. Walls of the base 51 are thin to reduce their resistance to breathing.

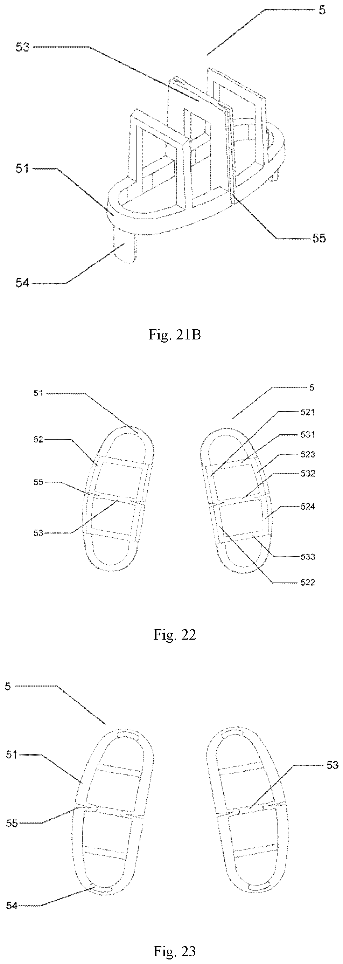

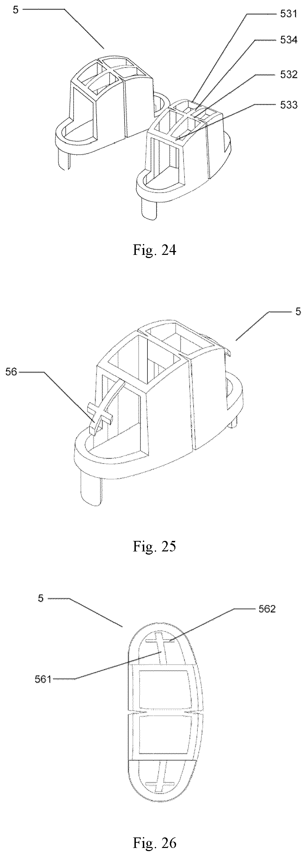

As shown in Figs. 21A, 21B, 22 and 41, the support beam 53 is a strip-like structure, which is located in a middle upper part of the bracket 5. The appearance of the support beam 53 is a straight-line, polyline, arc-line, arch, downward-open rectangle, downward-open trapezoid shape, or other common shape or irregular shape. The support beam 53 has two ends connected to the top of the base 51 or other remaining support beams, and together with the spoke 32, clips and fixes the fixed portion 44 of the filter plate 4 in both directions from below and above. As shown in Figs. 10 and 22, a first spoke 321 and a first support beam 531, a second spoke 322 and a second support beam 532, and a third spoke 323 and a third support beam 533 respectively clip and fix corresponding parts of the fixed portion 44 in a one-to-one correspondence manner. As shown in Figs. 24 and 41, preferably, a plurality of support beams 53 can cross each other and their intersecting parts can be fused integrally to form a grid-like structure.

As shown in Figs. 21A, 21B and 22, preferably, the bracket 5 further includes at least one side wall 52. The side wall 52 is a vertical flat plate wall or arc-shaped plate wall-like structure, which is located in the upper middle part of the bracket 5. The side wall 52 is vertically erected on the base 51, and has a bottom end fixed to the base 51, a top end located directly below the corresponding sealing edge 33, and two sides connected to the support beam 53. The side wall 52 and the sealing edge 33 clip and fix an edge part of the fixed portion 44 of the filter plate 4 in both directions from below and above. In the case a side wall 52 is provided, as shown in Figs. 10 and 22, a first sealing edge 331 and a first side wall 521, a second sealing edge 332 and a second side wall 522, a third sealing edge 333 and a third side wall 523, and a fourth sealing edge 334 and a fourth side wall 524 respectively clip and fix edges of the fixed portion 44 at that position in a one-to-one correspondence manner.

As shown in Figs. 21A and 23, preferably, the bracket 5 is also provided with at least one snap 54. The snap 54 is a vertical strip-like or sheet-like structure, and is positioned below an edge part of a lower surface of the base 51. The snap 54 is used to assist a wearer to detach the bracket 5 from the outer frame 3. The snap 54 has an upper end integrally connected to an edge part of a lower surface of the base 51, and a main body protruding downward from the lower surface of the base 51. In disassembly, a wearer pulls the snap 54 toward the direction of the nasal passage axis 97, causing the outer circumferential wall of the base 51 there to form a gap with the inner wall of the outer frame 3, such that the bracket 5 is no longer constrained by the annular frame 31 and the clamping tooth 35, and is detached smoothly.

As shown in Figs. 21A and 21B, preferably, the side face of the bracket 5 is also longitudinally provided with at least one groove-like fixing slot 55. The fixing slot 55 cooperates with the fixing rail 34 of the outer frame 3 to fix the bracket 5.

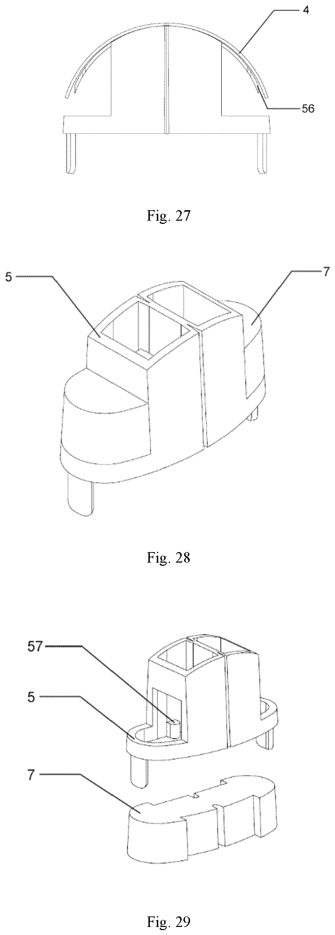

As shown in Figs. 25, 26 and 27, preferably, an upper part of the bracket 5 is also provided with at least one elastic carrier 56, which is used to assist the openable-closable portion 45 to recover to the closed state. The elastic carrier 56 includes at least one elastic strip 561 and at least one optional carrier strip 562. The elastic strip 561 is a strip made of an elastic material, the appearance of which is a straight-line, polyline, arc-line, or arch shape. The elastic strip 561 has one end integrally or detachably fixed to the support beam 53, a main body extending from the fixed part toward a direction away from the nasal passage axis 97, and the other end not fixed. The carrier strip 562 is a transversely arranged strip, which is fixed to the elastic strip 561. As shown in Fig. 27, after assembly, the elastic carrier 56 is positioned below the openable-closable portion 45 of the filter plate 4, and an upper surface thereof is tightly fit to or close to a lower surface of the openable-closable portion 45. In exhalation, the openable-closable portion 45 is pushed into the open state by an expiratory airflow and pushes the elastic carrier 56 to bend downward. After the end of exhalation, a resilience force of the elastic carrier 56 can cause the openable-closable portion 45 to recover to the closed state more quickly.

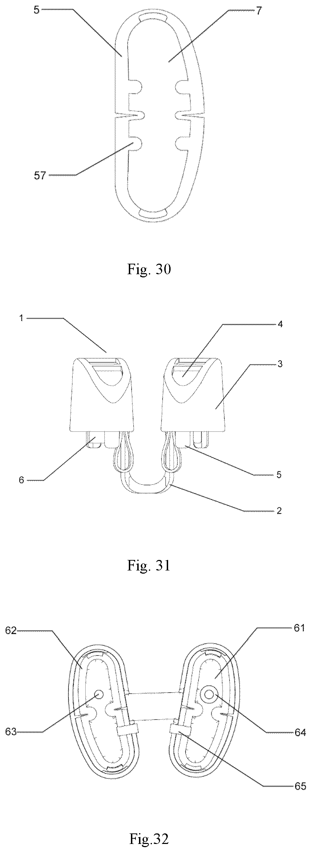

As shown in Figs. 28, 29 and 30, preferably, an inner cavity of each filtering mechanism is also provided with a block-like filter body 7, and the filter bodies 7 of the two filtering mechanisms are arranged symmetrically. The filter body 7 is made of a filter material, and is used to strengthen the filtering ability of the filtering mechanism. As shown in Fig. 29, in assembly, the filter body 7 is put into a cavity of the bracket 5 from an opening below the bracket 5. Preferably, the filter body 7 is an elastomer so as to be easier to mount into the bracket 5, and as an elastomer, the filter body 7 positioned below the filter plate 4 does not affect the filter plate 7 entering into the open state, and the resilience force of the filter body 7 itself can also be used to assist the filter plate 4 to quickly recover to the closed state after the end of exhalation. As shown in Fig. 30, as an optimization, to better fix the filter body 7, at least one fixing tooth 57 is integrally provided on an inner wall of a lower end of the bracket 5. The fixing tooth 57 is a block, a sheet or a strip, with its side surface being integrally connected to the inner wall of a lower part of the bracket 5, and its main body protruding from the inner wall of the bracket 5 toward the direction of the nasal passage axis 97. After the elastic filter body 7 is assembled in place, the fixing tooth 57 clamps the filter body 7 from below so that it is not liable to escape from the bracket 5. As an optimization, the filter body 7 can be a stacked multi-layer structure, including filter layers with different functions, each layer being made of a filter material with a different efficiency or for a different filtration object, and a plurality of filter layers are used in combination to meet higher filtration requirements.

As shown in Figs. 31, 32, 33, 34, 35, 36A and 36B, preferably, each filtering mechanism is also provided with an openable-closable waterproof stopper 6, and the waterproof stoppers 6 of the two filtering mechanisms are arranged symmetrically. The waterproof stopper 6 has two states: open and closed states. When a wearer is swimming, diving or performing other water activity, the waterproof stopper 6 is set to the closed state, such that it can block the influx of water into the nasal passages 95. When the wearer stops the water activity, the waterproof stopper 6 is set to the open state, such that the wearer can breathe normally with the nose.

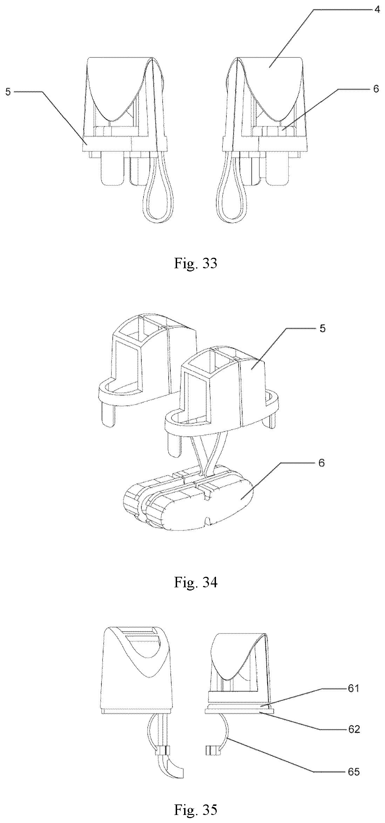

As shown in Figs. 32 and 36A, the waterproof stopper 6 includes a stopper body 61, at least one skirt 62 and at least one connecting band 65. The stopper body 61 is a column structure, the shape and size of a transverse cross-section of the top of which are same as the shape and size of a transverse cross-section of an inner cavity at a lower end of the bracket 5. As shown in Fig. 33, in order to set the waterproof stopper 6 to the closed state, a wearer inserts the stopper body 61 into the lower end of the bracket 5 from the bottom up, and the stopper body 61 is fixed inside the bracket 5 by using friction generated after they are tightly engaged with each other. At that point, the stopper body 61 completely closes an opening at the lower end of the bracket 5, such that water cannot enter the nasal passages 95. After the wearer removes the stopper body 61 from the bracket 5, the waterproof stopper 6 is set to the open state, such that the wearer can breathe normally.

As shown in Figs. 32 and 36A, the skirt 62 is a ring-like structure or discontinuous ring-like structure, which surrounds a lower part of the stopper body 61 and is integrally connected to the stopper body 61. When the waterproof stopper 6 is in the closed state, the skirt 62 clamps the underside of the bracket 5 to prevent the stopper body 61 from entering deeper into the inner cavity of the bracket 5 and thereby being difficult to take out. In addition, the skirt 62 can also improve the airtight effect at the junction between the waterproof stopper 6 and the bracket 5.

As shown in Figs. 32 and 36A, the connecting band 65 is a band-like structure, one end of which is integrally or detachably connected to the waterproof stopper 6, and the other end of which is integrally or detachably connected to the connecting beam 2, the outer frame 3 or the bracket 5.

As shown in Fig. 35, in other specific embodiments, the waterproof stopper 6 includes a stopper body 61, at least one skirt 62 and at least one connecting band 65. The stopper body 61 is still a column structure, the shape and size of a transverse cross-section of the top of which are same as the shape and size of a transverse cross-section of an inner cavity at a lower end of the annular frame 31. In order to set the waterproof stopper 6 to the closed state, a wearer inserts the stopper body 61 into the lower end of the annular frame 31 from the bottom up, and the stopper body 61 is fixed inside the annular frame 31 by using friction generated after they are tightly engaged with each other. At that point, the stopper body 61 completely closes the opening at the lower end of the outer frame 3, such that water cannot enter the nasal passages 95. After the wearer removes the stopper body 61 from the outer frame 3, the waterproof stopper 6 is set to the open state, such that the wearer can breathe normally. The skirt 62 is a ring-like structure or discontinuous ring-like structure, which surrounds a lower part of the stopper body 61 and is integrally connected to the stopper body 61. When the waterproof stopper 6 is in the closed state, the skirt 62 is clamped at the underside of the annular frame 31 to prevent the stopper body 61 from entering deeper into the inner cavity of the outer frame 3 and thereby being difficult to take out. In addition, the skirt 62 can also improve the airtight effect at the junction between the waterproof stopper 6 and the annular frame 31. The connecting band 65 is a band-like structure, one end of which is integrally or detachably connected to the waterproof stopper 6, and the other end of which is integrally or detachably connected to the connecting beam 2 or the outer frame 3.

As shown in Figs. 32, 36A and 36B, preferably, the symmetrically arranged two waterproof stoppers 6 are also provided with storage devices, wherein one waterproof stopper 6 is provided with at least one columnar storage plug 63 integrally connected therewith and protruding from a lower surface thereof, and the other waterproof stopper 6 is provided with at least one tubular storage slot 64 integrally connected therewith and protruding from a lower surface thereof, the storage plug 63 and the storage slot 64 being provided in pair and being matched with each other. As shown in Figs. 36A and 36B, when the two waterproof stoppers 6 are both in the open state, the storage plug 63 provided on the one waterproof stopper 6 is inserted into the storage slot 64 provided on the other waterproof stopper 6, and the former is tightly engaged with the latter, and by using friction therebetween, the two waterproof stoppers 6 are connected under the nasal septum 91 to avoid shaking of the waterproof stoppers 6, which would otherwise cause discomfort to the wearer.

In summary, the filtering mechanism of the nasal filter 1 of the present invention has the following advantages:
1. It is applicable to a variety of scenarios. First, the filter plate 4 and filter body 7 can filter out suspended particulate matters, suspended droplets or harmful gases in air. Second, during surfing, boating and other water sports, the filter plate 4 can block splashing water droplets to avoid the water droplets being splashed into the nasal passages 95 to cause rhinitis or nasosinusitis. When the water droplets slide off the filter plate 4, a wearer can breathe smoothly again, so there is no need to use a traditional swimming nasal clip to close nasal passages 95 for a long time. Third, during swimming, diving and other water sports, the waterproof stopper 6 can avoid the influx of water into the nasal passage 95. To sum up, for people on vacation at the seaside and lakeshore, at transition between water surface sports and underwater sports, they can switch between different usage modes by opening or closing the waterproof stopper 6 to achieve an optimum protection effect and user experience.
2. The filter plate 4 is in a convex-face or serrated-face form, which increases the air permeable area of the filtering mechanism, and significantly improves the air permeability and comfort of the nasal filter 1.
3. The filtering mechanism has a unidirectional filtering function, which improves the air permeability and comfort for a wearer during an exhalation phase.
4. The filtering mechanism is arranged inside nasal passages 95, and thus is more invisible and aesthetically pleasing compared with a mask. In the case of waterproof use, the nasal filter 1 of the present invention does not damage the skin outside the nose and has less resistance during swimming compared with a swimming nasal clip.
5. It is compact, stable and firm in structure, and easy to assemble and disassemble. It allows convenient replacement of the filter plate 4 and the filter body 7 with those of different types and efficiencies, and thus is more versatile. For a single use, it only needs to replace the filter plate 4 and the filter body 7 periodically, while other components can be reused, so it is more economical.

### Embodiment 1.

Figs. 2, 3, 4, 5, 6, 7, 8A, 8B, 9, 10, 11, 12, 13, 14, 15, 16, 17, 18, 21A, 21B, 22, 23, 24, 25, 26, 27, 28, 29, 30, 31, 32, 33, 34, 36A and 36B illustrate a first preferred embodiment of the present invention. As shown in Fig. 2, the nasal filter 1 includes a connecting beam 2 and two filtering mechanisms. Two ends of the connecting beam 2 are respectively integrally connected to the two filtering mechanisms that are arranged symmetrically. As shown in Figs. 8A and 8B, when worn, the two filtering mechanisms are respectively inserted into the two nasal passages 95 from the bottom up, and the connecting beam 2 semi-surrounds a lower end of a nasal septum 91.

As shown in Figs. 2, 5, 8A and 8B, the connecting beam 2 is a U-shaped structure. The connecting beam 2 includes an intermediate portion 21 positioned below the nasal septum 91 and two connecting arms 22 positioned on two sides of the nasal septum 91. The intermediate portion 21 is positioned between the two connecting arms 22. The two connecting arms 22 are respectively connected to the two filtering mechanisms arranged symmetrically in the two nasal passages 95.

The connecting beam 2 also provides a fixing function for the nasal filter 1. When worn, a minimum distance between the two connecting arms 22 is less than a width of the nasal septum 91 there, so that they apply a clipping force to the nasal septum 91. The clipping force is applied to the two sides of the nasal septum 91 directly by the two connecting arms 22 of the connecting beam 2.

As shown in Figs. 2 and 5, the two filtering mechanisms are symmetrically connected to the connecting beam 2, and each filtering mechanism includes an outer frame 3, a filter plate 4 and a bracket 5. As shown in Fig. 3, in assembly, first the filter plate 4 is mounted into the outer frame 3 from the bottom up, and then the bracket 5 is mounted and fixed to an inner side of the outer frame 3 from the bottom up. After assembly is finished, the outer frame 3 and the bracket 5 sleeved in its inner cavity fix the filter plate 4 in both directions from above and below, i.e. the filter plate 4 is positioned between the outer frame 3 and the bracket 5.

As shown in Figs. 9 and 10, the outer frame 3 includes an annular frame 31, 3 spokes 32, 6 sealing edges 33, two fixing rails 34 and two clamping teeth 35. The annular frame 31 forms a side wall of the outer frame 3, and the spokes 32 and the sealing edges 33 form the top of the outer frame 3.

As shown in Figs. 9 and 10, the annular frame 31 is a hollow ring-like structure open at two ends, the appearance of which is an irregular truncated cone with a small top and a large bottom. When worn, an axis of the annular frame 31 is parallel to a nasal passage axis 97, and an outer circumferential wall of the annular frame 31 is fit to or tightly engaged with an inner wall 96 of the nasal passage, and their interaction forces fix the nasal filter 1 in the nasal passage 95. An inner cavity of the annular frame 31 is through in the direction of the nasal passage axis 97, such that inspiratory and expiratory airflows can pass smoothly through the annular frame 31. Walls of the annular frame 31 are thin to reduce their resistance to breathing.

As shown in Fig. 9, the 3 spokes 32 and the 6 sealing edges 33 together form the top of the outer frame 3. The top is a smooth convex-face structure, which matches the filter plate 4 of the same shape below, so that the filtering area of the filtering mechanism is larger than that of a filtering mechanism whose outer frame 3 has a flat top, thereby improving the air permeability and comfort of the nasal filter 1.

As shown in Fig. 9, the 3 spokes 32 are all straight-line strip-like structures, specifically a first spoke 321, a second spoke 322 and a third spoke 323. The 3 spokes are arranged parallel to each other on the top of the outer frame 3, and two ends of each spoke are integrally connected to different positions on an inner wall of the top of the annular frame 31. The spokes 32 and a support beam 53 of the bracket 5 clip and are fixed to the filter plate 4 in both directions from above and below. The spoke 32 also constrain the filter plate 4 and the bracket 5 below the outer frame 3 to avoid a risk of being sucked in. As shown in Fig. 11, as an optimization, the outer frame 3 is also provided with a fourth spoke 324, which intersects with the first spoke 321, the second spoke 322 and the third spoke 323, and intersecting parts are integrally fused to form a grid-like structure. As shown in Fig. 12, as an optimization, the top of the outer frame 3 is also provided with two small spokes 37, wherein two ends of one small spoke 37 are respectively connected to middle segments of the first spoke 321 and the second spoke 322 that are arranged parallel to each other; and one end of the other small spoke 37 is connected to a middle segment of the third spoke 323, and the other end thereof is connected to the inner wall of the top of the annular frame 31. The small spokes 37 are used to assist the spokes 32 to achieve a function of constraining and fixing the filter plate 4.

As shown in Fig. 10, the 6 sealing edges 33 are straight-line or arc-line strip-like structures, specifically: a first sealing edge 331, a second sealing edge 332, a third sealing edge 333, a fourth sealing edge 334, a fifth sealing edge 335 and a sixth sealing edge 336. The 6 sealing edges 33 are provided along an edge of the top of the annular frame 31, and completely cover contact parts of the filter plate 4 and the annular frame 31, to avoid leakage at an edge part where the filter plate 4 contacts the annular frame 31. An outer side of each sealing edge 33 is integrally connected to the top of the annular frame 31, and a main body thereof protrudes from an inner wall of the annular frame 31 toward the direction of the axis of the annular frame 31. Two ends of the sealing edge 33 are integrally connected to roots of the spokes 32. For example, two ends of the first sealing edge 331 are respectively connected to roots of the first spokes 321 and the second spokes 322, and two ends of the fifth sealing edge 335 are respectively connected to two roots of the first spoke 321.

As shown in Figs. 9 and 10, the two fixing rails 34 are longitudinally provided on opposite sides of the annular frame 31. The fixing rails 34 are vertical trigonal prisms, which are used to fix the filter plate 4 and the bracket 5. A side surface of each fixing rail 34 is integrally connected to the inner wall of the annular frame 31, and a main body thereof protrudes from the inner wall of the annular frame 31 toward the axis of the annular frame 31 to form a trigonal prism structure. The bottom of the fixing rail 34 is provided with an arc-shaped chamfer to facilitate installation of the filter plate 4 and the bracket 5. As shown in Figs. 3, 13, 21A and 21B, two groove-shaped clamping slots 4 are longitudinally provided at corresponding positions on two sides of the filter plate 4, and two groove-shaped fixing slots 55 are longitudinally provided at corresponding positions on two sides of the bracket 5. In assembly, the clamping slots 43 and the fixing slots 55 are aligned with the fixing rail 34, and then the filter plate 4 and the bracket 5 are successively put into the outer frame 3 along the fixing rail 34. After assembly, the clamping slots 43 and the fixing slots 55 are tightly engaged with the fixing rail 34, thereby avoiding lateral or rotational displacement of the filter plate 4 and the bracket 5 inside the outer frame 3.

As shown in Fig. 10, two arc-line strip-like clamping teeth 35 are provided transversely on inner walls of front and rear ends close to a lower end of the annular frame 31. A side surface of each clamping tooth 35 is integrally connected to the inner wall of the annular frame 31, and a main body thereof protrudes from the inner wall of the annular frame 31 toward the direction of the axis of the annular frame 31 to form an arc-line strip-like structure. In assembly, after the bracket 5 is inserted into the cavity of the annular frame 31 from its bottom end, an outer circumferential wall of a base 51 is tightly fit to the inner wall of the annular frame 31, and an upper surface of the clamping tooth 35 clamps an edge of a lower surface of the base 51 so that the bracket 5 cannot escape toward the underside of the outer frame 3.

As shown in Figs. 2, 3 and 13, the filter plate 4 is a smooth convex-shaped sheet, which is positioned below the spokes 32 and the sealing edges 33 and is fixed between the outer frame 3 and the bracket 5. In a natural state, the filter plate 4 completely covers an opening above the annular frame 31, and an upper surface and a side face thereof are tightly fit to the inner wall of the outer frame 3, so that an airtight state is maintained between the filter plate 4 and the outer frame 3. The convex-face structure of the filter plate 4 can significantly increase its air permeable area, thereby improving the air permeability and comfort of the nasal filter 1. As shown in Fig. 13, two groove-shaped clamping slots 43 are also longitudinally provided on two sides of the filter plate 4, and the clamping slots 43 cooperate with the two fixing rails 34 on the outer frame 3 to fix the filter plate 4. The filter plate 4 is an elastomer to assist the filtering mechanism to achieve a unidirectional filtering function.

As shown in Fig. 13, the filter plate 4 includes a filter layer body 41 and an elastic layer 42 that are stacked. The filter layer body 41 is a single-layer structure for filtering suspended particulate matters, suspended droplets, splashing water droplets or harmful gases in air. As shown in Fig. 14, as an optimization, the filter layer body 41 is a stacked double-layer structure, containing a first filter layer body 411 and a second filter layer 412 with different functions. The first filter layer body 411 and the second filter layer body 412 are of the same shape and size, and are bonded to each other. The first filter layer body 411 is made of a medium-effect filter material, and the second filter layer body 412 is made of an activated carbon filter material. Suspended particulate matters and harmful gases can be filtered out at the same time by using the two filter layers in combination. As a further optimization, the filter layer body 41 is a stacked multi-layer structure, and a plurality of filter layers are used in combination to meet higher filtration requirements.

As shown in Fig. 15, the elastic layer 42 is positioned below the filter layer body 41, and they are not bonded to each other. The shape and size of the elastic layer 42 are same as the shape and size of the filter layer body 41. The elastic layer 42 is a grid-like structure made of an elastic material, for improving the elasticity of the filter plate 4, thereby assisting the filtering mechanism to achieve a unidirectional filtering function.

The filtering mechanism of this embodiment has a unidirectional filtering function, specifically implemented as follows. As shown in Figs 16, 17 and 18, the filter plate 4 includes a fixed portion 44 and two openable-closable portions 45. The fixed portion 44 is located in the middle of the filter plate 4. After assembly, the fixed portion 44 is tightly clipped by the outer frame 3 positioned above and the bracket 5 positioned below, such that it does not displace inside the outer frame 3. The openable-closable portions 45 are located at edge parts of two sides of the filter plate 4, and are inclined downwards in a direction away from the nasal passage axis 97 with an angle of inclination increasing progressively, so that the openable-closable portions 45 have a downward-tilted arc-shaped face to increase the air permeation efficiency of the openable-closable portions 45 in an open state. The openable-closable portions 45 are constrained above by the outer frame 3 but are not constrained below by the bracket 5, so when the openable-closable portions 45 are in a closed state, once the openable-closable portions 45 are subjected to a top-down acting force, they can move downward and open from a closed position of being closely fit to lower surfaces of the spokes 32 and the sealing edges 33.

As shown in Fig. 18, in a natural state and an inhalation state, the openable-closable portions 45 are in the closed state, with their upper surfaces being closely fit to the lower surfaces of the spokes 32 and the sealing edges 33, and an airtight state is maintained between the filter plate 4 and the outer frame 3, such that an inspiratory airflow can only pass through the filter plate 4, and the filtering mechanism performs the unidirectional filtering function. In an exhalation state, under the action of a larger air pressure above, the openable-closable portions 45 move downward to a position of openable-closable portions 45' in the open state; at that point, a gap is generated between the filter plate 4 and the outer frame 3, and most of an expiratory airflow passes through the gap, and the filtering mechanism does not perform the filtering function; and after the end of the exhalation state, under the action of the resilience of the filter plate 4, the openable-closable portions 45 recover to the closed state in the natural state.

The unidirectional filtering function has the beneficial effect that the expiratory airflow does not pass through the filter plate 4, but passes directly through the gap between the filter plate 4 and the outer frame 3, which greatly reduces exhalation resistance, thereby significantly improving the air permeability and comfort of the nasal filter 1 in the exhalation state.

As shown in Figs. 2, 3, 21A and 21B, the bracket 5 is sleeved in an inner cavity of the outer frame 3, and the bracket 5 is positioned below the filter plate 4, and includes a base 51, 4 side walls 52, 3 support beams 53, two snaps 54 and two fixing slots 55. An upper surface of the top of the bracket 5 is a smooth convex face.

As shown in Figs. 21A, 21B, 22 and 23, the base 51 is located in the lower part of the bracket 5, and is a ring-like structure. After assembly, an axis of the base 51 is parallel to the axis of the annular frame 31, and its outer circumferential wall is tightly engaged with the inner wall of the annular frame 31, and their interaction forces fix the bracket 5 in the inner cavity of the outer frame 3. An inner cavity of the base 51 is through in the direction of the nasal passage axis 97, and inspiratory and expiratory airflows can pass smoothly through its inner cavity. Walls of the base 51 are thin to reduce their resistance to breathing.

As shown in Figs. 21A, 21B and 22, the 3 support beams 53 are strip-like structures, the appearance of which is a downward-open trapezoid shape, and the 3 support beams 53 are located in an upper middle part of the bracket 5, specifically: a first support beam 531, a second support beam 532 and a third support beam 533. The support beams 53 each have two ends connected to an upper surface of the top of the base 51, and together with the spokes 32, clip and fix the fixed portion 44 of the filter plate 4 in both directions from below and above. As shown in Figs. 10 and 22, the first spoke 321 and the first support beam 531, the second spoke 322 and the second support beam 532, and the third spoke 323 and the third support beam 533 respectively clip and fix corresponding parts of the fixed portion 44 in a one-to-one correspondence manner. As shown in Fig. 24, as an optimization, the bracket 5 is also provided with a fourth support beam 534, two ends of which are integrally connected to middle segments of the first support beam 531 and the third support beam 533. The fourth support beam 534 also intersects with the second support beam 532, and their intersecting parts are integrally fused. Finally, the 4 support beams 53 are fused into a grid-like structure.

As shown in Figs. 21A, 21B and 22, the 4 side walls 52 are vertical flat plate walls or arc-shaped plate wall-like structures, specifically: a first side wall 521, a second side wall 522, a third side wall 523 and a fourth side wall 524. The side walls 52 are located in an upper middle part of the bracket 5, and each have a bottom end fixed to the base 51, a top end located directly below the corresponding sealing edge 33, and two sides connected to the support beam(s) 53. The side walls 52 and the sealing edges 33 clip and fix edge parts of the fixed portion 44 of the filter plate 4 in both directions from below and above. As shown in Figs. 10 and 22, a first sealing edge 331 and a first side wall 521, a second sealing edge 332 and a second side wall 522, a third sealing edge 333 and a third side wall 523, and a fourth sealing edge 334 and a fourth side wall 524 respectively clip and fix edges of the fixed portion 44 at corresponding positions in a one-to-one correspondence manner.

As shown in Figs. 21A and 23, the two snaps 54 are vertical sheet-like structures, which are respectively integrally connected to lower surfaces of front and rear ends of the base 51, and main bodies of the snaps 54 protrude downward from the lower surfaces of the base 51. The snaps 54 are used to assist a wearer to detach the bracket 5 from the outer frame 3. In disassembly, a wearer pulls the two snaps 54 toward the direction of the nasal passage axis 97 at the same time, causing outer walls of the front and rear ends of the base 51 to form a gap with the inner wall of the outer frame 3, such that the bracket 5 is no longer constrained by the annular frame 31 and the two clamping teeth 35, and is detached smoothly.

As shown in Figs. 21A and 21B, the two fixing slots 55 are groove structures, which are longitudinally provided on two sides of the second support beam 532, and the fixing slots 55 cooperate with the fixing rails 34 to fix the bracket 5.

As shown in Figs. 25, 26 and 27, as an optimization, an upper part of the bracket 5 is also provided with two elastic carriers 56, which are used to assist the openable-closable portions 45 to recover to the closed state. Each elastic carrier 56 includes an elastic strip 561 and an optional carrier strip 562, and the elastic strip 561 is an arc-line strip made of an elastic material. The two elastic strips 561 each have one end integrally fixed to outer sides of the first spoke 321 and the third spoke 323, a main body extending from the fixed part toward a direction away from the nasal passage axis 97, and the other end not fixed. The carrier strip 562 is a transversely arranged straight-line strip, which crosses the elastic strip 561 and is integrally connected thereto at an intersection. As shown in Fig. 27, after assembly, the elastic carrier 56 is positioned below the openable-closable portion 45, and an upper surface thereof is tightly fit to a lower surface of the openable-closable portion 45. In exhalation, the openable-closable portion 45 is pushed into the open state by an expiratory airflow and pushes the elastic carrier 56 to bend downward. After the end of exhalation, a resilience force of the elastic carrier 56 can cause the openable-closable portion 45 to recover to the closed state more quickly.

As shown in Figs. 28, 29 and 30, as optimization, each filtering mechanism is also provided with a block-like filter body 7, and the filter bodies 7 of the two filtering mechanisms are arranged symmetrically. The filter body 7 is an elastomer made of a filter material, and is used to strengthen the filtering ability of the filtering mechanism. As shown in Fig. 29, in assembly, the filter body 7 is put into a lower middle part of a cavity of the bracket 5 from an opening below the base 51. After assembly, the filter body 7 is fixed in a cavity enclosed by the base 51, the side walls 52 and the support beams 53, wherein the support beams 53 are positioned above and at outer sides of the filter body 7, the side walls 52 are located at outer sides of the filter body 7, the base 51 is positioned below the filter body 7, and the front and rear ends of the base 51 clamp the filter body 7 from below so that it is not liable to escape from the bracket 5. As an elastomer, the filter body 7 also helps in implementing the unidirectional filtration of the filtering mechanism. After assembly, the filter body 7 is positioned below the filter plate 4. In exhalation, the openable-closable portions 45 open downwards, and push an upper surface of the filter body 7 and cause the filter body 7 to deform elastically. After the end of exhalation, a resilience force causes the filter body 7 to quickly recover to its original state, and also assists the openable-closable portions 7 to recover to the closed state more quickly. As shown in Fig. 30, 4 fixing teeth 57 are integrally provided on an inner wall of a lower end of the base 51. The fixing teeth 57 each have a side surface integrally connected to the inner wall of the base 51, and a main body protruding from the inner wall of the base 51 toward the direction of the axis of the base 51. After the elastic filter body 7 is assembled in place, the fixing teeth 57 clamp the filter body 7 from below so that it is not liable to escape from the bracket 5. As an optimization, the filter body 7 is stacked multi-layer structure, including filter layers with different functions, each layer being made of a filter material with a different efficiency or for a different filtration object, and a plurality of filter layers are used in combination to meet higher filtration requirements.

As shown in Figs. 31, 32, 33 and 34, as an optimization, each filtering mechanism is also provided with an openable-closable waterproof stopper 6, and the waterproof stoppers 6 of the two filtering mechanisms are arranged symmetrically. The waterproof stopper 6 has two states: open and closed states. When a wearer is swimming, diving or performing other underwater activity, the waterproof stopper 6 is set to the closed state, such that it can block the influx of water into the nasal passages 95. When the wearer stops the underwater activity, the waterproof stopper 6 is set to the open state, such that the wearer can breathe normally with the nose.

As shown in Figs. 32 and 36A, the waterproof stopper 6 includes a stopper body 61, a skirt 62, a storage plug 63, a storage slot 64 and a connecting band 65.

The stopper body 61 is a flat column structure, the shape and size of a transverse cross-section of the top of which are same as the shape and size of a transverse cross-section of an inner cavity at a lower end of the base 51. As shown in Fig. 33, in order to set the waterproof stopper 6 to the closed state, a wearer inserts the stopper body 61 into the lower end of the base 51 from the bottom up, and the stopper body 61 is fixed to a lower part of the base 51 by using friction generated after they are tightly engaged with each other. At that point, the stopper body 61 completely closes an opening at the lower end of the bracket 5, such that water cannot enter the nasal passages 95. After the wearer removes the stopper body 61 from the bracket 5, the waterproof stopper 6 is set to the open state, such that the wearer can breathe normally.

As shown in Figs. 32 and 36A, the skirt 62 is a discontinuous ring-like structure, which surrounds a lower part of the stopper body 61 and is integrally connected to the stopper body 61. When the waterproof stopper 6 is in the closed state, the skirt 62 is clamped at the underside of the base 51 to prevent the stopper body 61 from entering deeper into the inner cavity of the bracket 5 and thereby being difficult to take out. In addition, the skirt 62 can also improve the airtight effect at the junction between the waterproof stopper 6 and the base 51.

As shown in Figs. 32 and 36A, the connecting band 65 is a band-like structure, one end of which is integrally connected to the waterproof stopper 6, and the other end of which is integrally connected to the bracket 5.

As shown in Figs. 32, 36A and 36B, one of the waterproof stoppers 6 is provided with a columnar storage plug 63 integrally connected therewith and protruding from a lower surface thereof, and the other waterproof stopper 6 arranged symmetrically with respect thereto is provided with a tubular storage slot 64 integrally connected therewith and protruding from a lower surface thereof. When the two waterproof stoppers 6 are in the open state, the storage plug 63 provided on the one waterproof stopper 6 is inserted into the storage slot 64 provided on the other waterproof stopper, and the former is tightly engaged with the latter, and by using friction therebetween, the two waterproof stoppers 6 are connected under the nasal septum 91 to avoid shaking of the waterproof stoppers 6, which would otherwise cause discomfort to the wearer.

The nasal filter 1 provided with the waterproof stoppers 6 is used for waterproofing, and the filter layer body 41 of the filter plate 4 cooperating therewith is usually a metal net or polyester fiber net with a pore size between 10 microns and 200 microns, and its specific use is as follows: When a wearer is surfing, boating or performing other water surface sports, the wearer sets the waterproof stoppers 6 to the open state, and at that time, the wearer can breathe normally. When water droplets are splashed into the nasal passages 95, the filter plate 4 can block the water droplets outside the nasal passage 95 by using surface tension of water to avoid entering the nasal passages 95 and inducing rhinitis and nasosinusitis. After the water droplets slide off the filter plate 4, the wearer resumes normal breathing. When the wearer is swimming, diving or performing other underwater sports, the wearer closes the waterproof stoppers 6 to avoid the influx of water into the nasal passages 95. Compared with a traditional swimming nasal clip, the nasal filter 1 of the present invention is applicable to a variety of water activity scenarios, and has the advantages of not harming the skin of the outer side of the nasal part, having low swimming resistance, etc.

### Embodiment 2.

Figs. 37, 38, 39, 40, 41 and 42 illustrate a second preferred embodiment of the invention. The basic structure of this embodiment is same as that of the first embodiment, and differs in that a filtering mechanism of this embodiment uses a serrated-face filter plate, which further increases the air permeable area of the filtering mechanism.

As shown in Fig. 37, the nasal filter 1 includes a connecting beam 2 and two filtering mechanisms. Two ends of the connecting beam 2 are respectively integrally connected to the two filtering mechanisms arranged symmetrically in nasal passages 95 on two sides.

As shown in Fig. 37, the connecting beam 2 is a U-shaped structure, which includes an intermediate portion 21 positioned below a nasal septum 91 and two connecting arms 22 positioned on two sides of the nasal septum 91. The intermediate portion 21 is positioned between the two connecting arms 22.

As shown in Fig. 37, the two filtering mechanisms are symmetrically connected to the connecting beam 2, and each filtering mechanism includes an outer frame 3, a filter plate 4 and a bracket 5.

As shown in Figs. 38 and 39, the outer frame 3 includes an annular frame 31, 6 spokes 32, 12 sealing edges 33, two fixing rails 34 and two clamping teeth 35. The annular frame 31 forms a side wall of the outer frame 3, and the spokes 32 and the sealing edges 33 form the top of the outer frame 3.

As shown in Figs. 38 and 39, the annular frame 31 is a hollow ring-like structure open at two ends, the appearance of which is an irregular truncated cone with a small top and a large bottom, and the top of the annular frame 31 is serrated. When worn, an axis of the annular frame 31 is parallel to a nasal passage axis 97, and an outer circumferential wall of the annular frame 31 is fit to or tightly engaged with an inner wall 96 of the nasal passage, and their interaction forces fix the nasal filter 1 in the nasal passage 95. An inner cavity of the annular frame 31 is through in the direction of the nasal passage axis 97, such that inspiratory and expiratory airflows can pass smoothly through the annular frame 31. Walls of the annular frame 31 are thin to reduce their resistance to breathing.

As shown in Figs. 38 and 39, the 6 spokes 32 and the 12 sealing edges 33 together form the top of the outer frame 3. The top of the outer frame 3 is a serrated-face structure, which matches the filter plate 4 of the same shape below, so that the filtering area of the filtering mechanism is larger than that of a filtering mechanism whose outer frame 3 has a flat top or convex-face top, thereby improving the air permeability and comfort of the nasal filter 1. Two ends of the 6 spokes 32 are connected to different positions of an inner wall of the top of the annular frame 31. A first spoke 321, a second spoke 322, a third spoke 323, a fourth spoke 324 and a fifth spoke 325 are straight-line strip-like structures arranged parallel to each other, which match a bent portion of the serrated-face structure of the top of the outer frame 3, wherein the first spoke 321, the third spoke 323 and the fifth spoke 325 are at higher positions, the second spoke 322 and the fourth spoke 324 are at lower positions, and the sixth spoke 326 is a polyline strip-like structure, which crosses the above-mentioned 5 spokes and is integrally connected thereto at intersections to form a grid-like structure.

As shown in Figs. 38 and 39, the 12 straight-line or arc-line strip-like sealing edges 33 are provided along an edge of the top of the annular frame 31. An outer side of each sealing edge 33 is integrally connected to the top of the annular frame 31, and a main body thereof protrudes from an inner wall of the annular frame 31 toward the direction of the axis of the annular frame 31. The sealing edges 33 each have two ends integrally connected to roots of the spokes 32, and completely cover contact parts of the filter plate 4 and the annular frame 31.

As shown in Figs. 38 and 39, the two fixing rails 34 are longitudinally provided on opposite sides of the annular frame 31, and are vertical trigonal prisms. A side surface of each fixing rail 34 is integrally connected to the inner wall of the annular frame 31, and a main body thereof protrudes from the inner wall of the annular frame 31 toward the axis of the annular frame 31 to form a trigonal prism structure. The bottom of the fixing rail 34 is provided with an arc-shaped chamfer to facilitate installation of the filter plate 4 and the bracket 5.

As shown in Fig. 39, two clamping teeth 35 are provided transversely on inner walls of front and rear ends close to a lower end of the annular frame 31, and are arc-line strip-like structures. A side surface of each clamping tooth 35 is integrally connected to the inner wall of the annular frame 31, and a main body thereof protrudes from the inner wall of the annular frame 31 toward the direction of the axis of the annular frame 31 to form an arc-line strip-like structure.

As shown in Fig. 40, the filter plate 4 is a serrated-face sheet, the shape of which matches a lower surface of the top of the outer frame 3. The filter plate 4 is positioned below the spokes 32 and sealing edges 33, and is fixed between the outer frame 3 and the bracket 5. In a natural state, the filter plate 4 completely covers an opening above the annular frame 31, and an upper surface and a side face thereof are tightly fit to the inner wall of the outer frame 3, so that an airtight state is maintained between the filter plate 4 and the outer frame 3. Compared with planar and convex-face structures, the serrated-face structure of the filter plate 4 can significantly increase the air permeable area of the filter plate 4, thereby improving the air permeability and comfort of the nasal filter 1. The filter plate 4 includes only a filter layer body 41. The filter layer body 41 is a single-layer structure for filtering suspended particulate matters, suspended droplets, splashing water droplets or harmful gases in air. Two groove-shaped clamping slots 43 are also longitudinally provided on two sides of the filter plate 4, and the clamping slots 43 cooperate with the two fixing rails 34 on the outer frame 3 to fix the filter plate 4. The filter plate 4 is an elastomer to assist the filtering mechanism to achieve a unidirectional filtering function.

The filtering mechanism of this embodiment has a unidirectional filtering function, specifically implemented as follows. As shown in Fig. 40, the filter plate 4 includes a fixed portion 44 and two openable-closable portions 45. The fixed portion 44 is a W-shaped sheet and is located in the middle of the filter plate 4. After assembly, the fixed portion 44 is tightly clipped by the outer frame 3 positioned above and the bracket 5 positioned below, such that it does not displace inside the outer frame 3. The openable-closable portions 45 are located at edge parts of two sides of the filter plate 4, and are inclined downwards in a direction away from the nasal passage axis 97 with an angle of inclination increasing progressively, so that the openable-closable portions 45 have a downward-tilted arc-shaped face to increase the air permeation efficiency of the openable-closable portions 45 in an open state. The openable-closable portions 45 are constrained above by the outer frame 3 but are not constrained below by the bracket 5, so when the openable-closable portions 45 are in a closed state, once the openable-closable portions 45 are subjected to a top-down acting force, they can move downward and open from a closed position of being closely fit to lower surfaces of the spokes 32 and the sealing edges 33. In a natural state and an inhalation state, the openable-closable portions 45 are in the closed state, with their upper surfaces being closely fit to the lower surfaces of the spokes 32 and the sealing edges 33, and an airtight state is maintained between the filter plate 4 and the outer frame 3, such that an inspiratory airflow can only pass through the filter plate 4, and the filtering mechanism performs the filtering function. In an exhalation state, under the action of a larger air pressure above, the openable-closable portions 45 move downward to the open state; at that point, a gap is generated between the filter plate 4 and the outer frame 3, and most of an expiratory airflow passes through the gap, and the filtering mechanism does not perform the filtering function; and after the end of the exhalation state, under the action of the resilience of the filter plate 4, the openable-closable portions 45 recover to the closed state in the natural state.

As shown in Figs. 37 and 41, the bracket 5 is sleeved in an inner cavity of the outer frame 3, and is positioned below the filter plate 4. The bracket 5 includes a base 51, 8 side walls 52, 6 support beams 53, two snaps 54 and two fixing slots 55. An upper surface of the top of the bracket 5 is a serrated face.

As shown in Fig. 41, the base 51 is located in a lower part of the bracket 5, and is a ring-like structure. After assembly, an axis of the base 51 is parallel to the axis of the annular frame 31, and an outer circumferential wall of the base 51 is tightly engaged with the inner wall of the annular frame 31, and their interaction forces fix the bracket 5 in a lower middle part of the inner cavity of the outer frame 3. An inner cavity of the base 51 is through in the direction of the nasal passage axis 97, and inspiratory and expiratory airflows can pass smoothly through its inner cavity. Walls of the base 51 are thin to reduce their resistance to breathing.

As shown in Fig. 41, the support beams 53 are located in a middle upper part of the bracket 5. A first support beam 531, a second support beam 532, a third support beam 533, a fourth support beam 534 and a fifth support beam 535 are strip-like structures, the appearance which is a downward-open trapezoid shape. Two ends of the above-mentioned 5 support beams 53 are integrally connected to an upper surface of the base 51. A sixth support beam 536 is a polyline strip-like structure, and two ends thereof are integrally connected to middle segments of the first support beam 531 and the fifth support beam 535, and the sixth support beam 536 crosses the second support beam 532, the third support beam 533 and the fourth support beam 534 and is integrally connected thereto at intersections to form a grid-like structure. The spokes 32 and the support beams 53 clip and fix the fixed portion 44 of the filter plate 4 in both directions from below and above. As shown in Figs. 39 and 41, the first spoke 321 and the first support beam 531, the second spoke 322 and the second support beam 532, the third spoke 323 and the third support beam 533, the fourth spoke 324 and the fourth support beam 534, the fifth spoke 325 and the fifth support beam 535, and the sixth spoke 326 and the sixth support beam 536 respectively clip and fix corresponding parts of the fixed portion 44 in a one-to-one correspondence manner.

As shown in Fig. 41, the 8 side walls 52 are vertical flat plate wall or arc-shaped plate wall-like structures, and are located in an upper middle part of the bracket 5. Each side wall 52 has a bottom end fixed to the base 51, a top end located directly below the corresponding sealing edge 33, and two sides connected to the support beam 53. The sealing edges 33 and the side walls 52 clip and fix edge parts of the fixed portion 44 of the filter plate 4 in both directions from below and above.

As shown in Fig. 41, the two snaps 54 are vertical sheet-like structures, which are respectively integrally connected to lower surfaces of front and rear ends of the base 51, and main bodies thereof protrude downward from the lower surfaces of the base 51. The snaps 54 are used to assist a wearer to detach the bracket 5 from the outer frame 3. In disassembly, a wearer pulls the two snaps 54 toward the direction of the nasal passage axis 97 at the same time, causing outer walls of the front and rear ends of the base 51 to form a gap with the inner wall of the outer frame 3, such that the bracket 5 is no longer constrained by the annular frame 31 and the two clamping teeth 35, and is detached smoothly.

As shown in Fig. 41, the two fixing slots 55 are groove-shaped structures, which are longitudinally provided on both sides of the third support beam 533, and the fixing slots 55 cooperate with the fixing rails 34 to fix the bracket 5.

As shown in Fig. 42, as an optimization, in order to avoid that a sharp part of the top of the annular frame 31 injures the inner wall 96 of the nasal passage, a ring-like protective cover 36 is also sleeved at an outer circumference of the outer frame 3. The protective cover 36 surrounds an upper part of the outer frame 3 from an outer side, with its lower end being connected to an upper end of the annular frame 31, and a top end of the protective cover 36 is not lower than the highest point of the annular frame 31.

Finally, it should be noted that the above embodiments are only used for describing instead of limiting the technical solutions of the present invention. Although the present invention is described in detail with reference to the embodiments, persons of ordinary skill in the art should understand that modifications or equivalent substitutions of the technical solutions of the present invention should be encompassed within the scope of the claims of the present invention so long as they do not depart from the spirit and scope of the technical solutions of the present invention.

## Claims

1. A nasal filter, wherein the nasal filter (1) is arranged within nasal passages, and comprises a connecting beam (2) and two filtering mechanisms; two ends of the connecting beam (2) are integrally or detachably connected to the two filtering mechanisms arranged symmetrically in the nasal passages (95) on two sides;
the connecting beam (2) semi-surrounds a lower end of a nasal septum (91), and comprises an intermediate portion (21) positioned below the nasal septum (91), and two connecting arms (22) arranged symmetrically on two sides of the nasal septum (91); the intermediate portion (21) is positioned between the two connecting arms (22); the two connecting arms (22) are connected to the two filtering mechanisms arranged symmetrically in the two nasal passages (95);
each filtering mechanism comprises an outer frame (3), a filter plate (4), and a bracket (5);
the outer frame (3) comprises an annular frame (31) and at least one spoke (32), the annular frame (31) being a hollow ring-like, elliptical columnar or tubular structure open at two ends and forming a side wall of the outer frame (3); when worn, an axis of the annular frame (31) is parallel to a nasal passage axis (97), an outer circumferential wall of the annular frame (31) is fit to an inner wall (96) of the nasal passage, and an inner cavity of the annular frame (31) is through in the direction of the nasal passage axis (97); the spoke (32) is a strip-like structure, two ends of which are integrally or detachably connected to the top of the annular frame (31), and the spoke (32) forms the top of the outer frame (3);
the sheet-like filter plate (4) is provided below the top of the outer frame (3); specifically, the filter plate (4) is positioned below the spoke (32), and completely covers an opening above the annular frame (31); the filter plate (4) comprises a fixed portion (44) and at least one openable-closable portion (45), the fixed portion (44) being located in the middle of the filter plate (4), the openable-closable portion (45) being located at an edge part of the filter plate (4), and the openable-closable portion (45) being not constrained by the bracket (5) below; and in an exhalation state, an expiratory airflow pushes the openable-closable portion (45) to switch from a closed state to an open state, so that the filtering mechanism has a unidirectional filtering function; and
the bracket (5) is sleeved in an inner cavity of the outer frame (3), and the bracket (5) is positioned below the filter plate (4), and the outer frame (3) and the bracket (5) constrain and fix the filter plate (4) from above and below.

2. The nose filter according to claim 1, wherein the outer frame (3) further comprises a sealing edge (33); the sealing edge (33) is a straight-line, polyline or arc-line-shaped strip-like structure, which is provided along an edge of the top of the annular frame (31); the sealing edge (33) is integrally connected to the top of the annular frame (31), and a main body thereof protrudes from an inner wall of the annular frame (31) toward the direction of the axis of the annular frame (31); and two ends of the sealing edge (33) are integrally connected to roots of the spoke (32).

3. The nasal filter according to claim 1, wherein at least one fixing rail (34) is also longitudinally provided at an inner side of the outer frame (3); the fixing rail (34) is a prism or a semi-circular cylinder whose axis is parallel to the axis of the annular frame (31); a side surface of the fixing rail (34) is integrally connected to an inner wall of the annular frame (31), and a main body thereof protrudes from the inner wall of the annular frame (31) toward the axis of the annular frame (31); and correspondingly, the filter plate (4) is longitudinally provided with groove-like clamping slots (43) on two sides to match therewith, and the bracket (5) is longitudinally provided with groove-like fixing slots (55) on two sides to match therewith.

4. The nasal filter according to claim 1, wherein the outer frame (3) is also provided with at least one strip-like or block-like clamping tooth (35); the clamping tooth (35) is provided on an inner wall close a lower end of the annular frame (31), with a side surface thereof being integrally connected to an inner wall of the annular frame (31), and a main body thereof protruding from the inner wall of the annular frame (31) toward the direction of the axis of the annular frame (31).

5. The nasal filter according to claim 1, wherein the filter plate (4) is an elastomer.

6. The nasal filter according to claim 1, wherein the filter plate (4) comprises a filter layer body (41) and an elastic layer (42) stacked with the filter layer body (41).

7. The nasal filter according to claim 6, wherein the filter layer body (41) is a stacked multi-layer structure, which comprises a plurality of layer structures made of filter materials with different filtering functions or filtering efficiencies.

8. The nasal filter according to claim 1, wherein the filter plate (4) is a convex-face or serrated-face sheet.

9. The nasal filter according to claim 1, wherein the openable-closable portion (45) is inclined downwards in a direction away from the nasal passage axis (97).

10. The nasal filter according to claim 9, wherein an angle of inclination of the openable-closable portion (45) inclined downwards in the direction away from the nasal passage axis (97) increases progressively.

11. The nasal filter according to claim 1, wherein the bracket (5) comprises a base (51) and at least one support beam (53);
the base (51) is a hollow ring-like, elliptical columnar or tubular structure open at two ends, and is located in a lower middle part of the bracket (5), and after assembly, an axis of the base (51) is parallel to the axis of the annular frame (31), and an outer circumferential wall thereof is tightly engaged with an inner wall of the annular frame (31); an inner cavity of the base (51) is through in the direction of the nasal passage axis (97);
the support beam (53) is a strip-like structure, the appearance of which is a straight-line, polyline, arc-line, arch, downward-open rectangle or downward-open trapezoid shape; the support beam (53) is located in an upper middle part of the bracket (5); the support beam (53) has two ends connected to the top of the base (51) or other remaining support beams, and together with the spoke (32), clips and fixes the fixed portion (44) of the filter plate (4) in both directions from below and above; and no support beam (53) is provided below the openable-closable portion (45).

12. The nasal filter according to claim 11, wherein the bracket (5) further comprises at least one side wall (52); the side wall (52) is a vertical flat plate wall or arc-shaped plate wall-like structure, which is located in the upper middle part of the bracket (5); the side wall (52) has a bottom end fixed to the base (51), a top end located directly below the corresponding sealing edge (33), and two sides connected to the support beam (53); and the side wall (52) and the sealing edge (33) clip and fix an edge part of the fixed portion (44) of the filter plate (4) in both directions from below and above.

13. The nasal filter according to claim 11, wherein the bracket (5) is also provided with at least one snap (54); the snap (54) is a vertical strip-like or sheet-like structure; and the snap (54) has an upper end integrally connected to an edge part of a lower surface of the base (51), and a main body protruding downward from the lower surface of the base (51).

14. The nasal filter according to claim 11, wherein the bracket (5) is also provided with at least one elastic carrier (56); the elastic carrier (56) comprises at least one elastic strip (561) and at least one optional carrier strip (562); the elastic strip (561) has one end integrally or detachably fixed to the support beam (53), a main body extending from the fixed part toward a direction away from the nasal passage axis (97), and the other end not fixed; the carrier strip (562) is a transversely arranged strip, which is fixed to the elastic strip (561); and after assembly, the elastic carrier (56) is positioned below the openable-closable portion (45), and an upper surface thereof is tightly fit to or close to a lower surface of the openable-closable portion (45).

15. The nasal filter according to claim 1, wherein the filtering mechanism further comprises a filter body (7); and after assembly, the filter body (7) is arranged in an inner cavity of the bracket (5).

16. The nasal filter according to claim 15, wherein the filter body (7) is an elastomer.

17. The nasal filter according to claim 15, wherein the filter body (7) is a stacked multi-layer structure, which comprises a plurality of layer structures made of filter materials with different filtering functions or filtering efficiencies.

18. The nasal filter according to claim 1, wherein the filtering mechanism further comprises a waterproof stopper (6); the waterproof stopper (6) comprises a stopper body (61), at least one skirt (62) and at least one connecting band (65);
the stopper body (61) is a column structure, the shape and size of a transverse cross-section of the top of which are same as the shape and size of a transverse cross-section of an inner cavity at a lower end of the bracket (5) or the annular frame (31), which enables the stopper body (61) to be plugged into and close the lower end of the bracket (5) or the annular frame (31); the skirt (62) is a ring-like structure or discontinuous ring-like structure, which surrounds a lower part of the stopper body (61) and is integrally connected to the stopper body (61); and the connecting band (65) is a band-like structure with one end integrally or detachably connected to the waterproof stopper (6) and the other end integrally or detachably connected to the connecting beam (2), the outer frame (3) or the bracket (5).

19. The nasal filter according to claim 18, wherein the two filtering mechanisms each comprise a waterproof stopper (6), respectively, wherein one waterproof stopper (6) is provided with at least one columnar storage plug (63) integrally connected therewith and protruding from a lower surface thereof, and the other waterproof stopper (6) is provided with at least one tubular storage slot (64) integrally connected therewith and protruding from a lower surface thereof, the storage plug (63) and the storage slot (64) being provided in pair and being matched with each other; and
when the two waterproof stoppers (6) are both in an open state, the two waterproof stoppers (6) are placed opposite each other, the storage plug (63) provided on the one waterproof stopper (6) is inserted into the storage slot (64) provided on the other waterproof stopper (6), and the former is tightly engaged with the latter, and by using friction therebetween, the two opposite waterproof stoppers (6) are connected under the nasal septum (91).

20. The nasal filter according to claim 1, wherein the connecting beam (2) applies a wrap-around force to the nasal septum (91); and the wrap-around force is applied directly to two sides of the nasal septum (91) by the two connecting arms (22) of the connecting beam (2), or after being conducted by the two connecting arms (22) of the connecting beam (2) to the corresponding filtering mechanisms, the wrap-around force is applied to the two sides of the nasal septum (91) by the corresponding filtering mechanisms.
